# EUROPEAN PATENT APPLICATION

(11) **EP 1 818 395 A1**
(43) Date of publication of application: **15.08.2007**
(21) Application number: 06290221.8
(22) Date of filing: 08.02.2006
(51) Int. Cl.: C12N 9/24, C07K 7/06, C07K 7/08, A61K 38/08, A61K 38/10, A61K 38/47, A61P 25/00

(54) **Compositions and methods for treating lysosomal storage diseases**

(71) Applicant: Diatos, 75014 Paris (FR)
(72) Inventor: Arranz, Valérie, 77122 Monthyon (FR)
(74) Representative: Breese Derambure Majerowicz

(57) **Abstract**

The invention relates to chimeric polypeptides comprising a lysosomal peptide fused or conjugated to at least one cell-penetrating peptide (CPP). Also provided by the invention are methods for treating a subject suffering from lysosomal storage disorders (LSD).

SEQUENCE LISTING

<110> DIATOS S.A.

<120> COMPOSITIONS AND METHODS FOR TREATING LYSOSOMAL STORAGE
DISEASES

<130> 46764/EP

<140> EP 06290221.8
<141> 2006-02-08

<160> 48

<170> PatentIn version 3.1

<210> 1

<211> 21

<212> PRT

<213> Bufo gargarizans

<220>
<223> Buforin II- Cell penetrating peptide

<400> 1

Thr Arg Ser Ser Arg Ala Gly Leu Gln Phe Pro Val Gly Arg Val
1               5                   10                  15

His Arg Leu Leu Arg Lys
                20

<210> 2

<211> 16

<212> PRT

<213> Homo sapiens

<220>
<223> DPV3-Cell penetrating peptide

<400> 2

Arg Lys Lys Arg Arg Arg Glu Ser Arg Lys Lys Arg Arg Arg Glu Ser
1               5                   10                  15

<210> 3

<211> 17

<212> PRT

<213> Homo sapiens

<220>
<223> DPV6-Cell penetrating peptide

<400> 3

Gly Arg Pro Arg Glu Ser Gly Lys Lys Arg Lys Arg Lys Arg Leu Lys Pro
1               5                   10                  15

<210> 4

<211> 15

<212> PRT

<213> Homo sapiens

<220>
<223> DPV7-Cell penetrating peptide

<400> 4

Gly Lys Arg Lys Lys Lys Gly Lys Leu Gly Lys Lys Arg Asp Pro
1               5                   10                  15

<210> 5

<211> 17

<212> PRT

<213> Homo sapiens

<220>
<223> DPV7b-Cell penetrating peptide

<400> 5

Gly Lys Arg Lys Lys Lys Gly Lys Leu Gly Lys Lys Arg Pro Arg Ser Arg
1               5                   10                  15

<210> 6

<211> 18

<212> PRT

<213> Homo sapiens

<220>
<223> DPV3/10-Cell penetrating peptide

<400> 6

Arg Lys Lys Arg Arg Arg Glu Ser Arg Arg Ala Arg Arg Ser Pro Arg His
1               5                   10                  15

Leu

<210> 7

<211> 19

<212> PRT

<213> Homo sapiens

<220>
<223> DPV10/6-Cell penetrating peptide

<400> 7

Ser Arg Arg Ala Arg Arg Ser Pro Arg Glu Ser Gly Lys Lys Arg Lys Arg
1               5                   10                  15

Lys Arg

<210>  8

<211>  19

<212>  PRT

<213> Homo sapiens

<220>
<223> DPV1047-Cell penetrating peptide

<400>  8

Val Lys Arg Gly Leu Lys Leu Arg His Val Arg Pro Arg Val Thr Arg Met
1               5                   10                  15

Asp Val

<210>  9

<211>  18

<212>  PRT

<213> Homo sapiens

<220>
<223> DPV1048-Cell penetrating peptide

<400>  9

Val Lys Arg Gly Leu Lys Leu Arg His Val Arg Pro Arg Val Thr Arg Asp
1               5                   10                  15

Val

<210>  10

<211>  14

<212>  PRT

<213> Homo sapiens

<220>
<223> DPV10-Cell penetrating peptide

<400> 10

Ser Arg Arg Ala Arg Arg Ser Pro Arg His Leu Gly Ser Gly
1               5                   10


<210> 11

<211> 16

<212> PRT

<213> Homo sapiens



<220>
<223> DPV15-Cell penetrating peptide

<400> 11

Leu Arg Arg Glu Arg Gln Ser Arg Leu Arg Arg Glu Arg Gln Ser Arg
1               5                   10                  15


<210> 12

<211> 22

<212> PRT

<213> Homo sapiens



<220>
<223> DPV15b-Cell penetrating peptide

<400> 12

Gly Ala Tyr Asp Leu Arg Arg Arg Glu Arg Gln Ser Arg Leu Arg
1               5                   10                  15

Arg Arg Glu Arg Gln Ser Arg
                20


<210> 13

<211> 30

<212> PRT

<213>  Artificial Sequence

<220>
<223>  GALA - Cell penetrating peptide

<400>  13

Trp Glu Ala Ala Leu Ala Glu Ala Leu Ala Glu Ala Leu Ala Glu
1               5                   10                  15

His Leu Ala Glu Ala Leu Ala Glu Ala Leu Glu Ala Leu Ala Ala
                20                  25                  30

<210>  14

<211>  21

<212>  PRT

<213>  Artificial Sequence

<220>

<223>  Cβ - Cell penetrating peptide

<400>  14

Lys Gly Ser Trp Tyr Ser Met Arg Lys Met Ser Met Lys Ile Arg
1               5                   10                  15

Pro Phe Phe Pro Gln Gln
                20

<210>  15

<211>  20

<212>  PRT

<213>  Artificial Sequence

<220>

<223> preCγ - Cell penetrating peptidde

<400>  15

Lys Thr Arg Tyr Tyr Ser Met Lys Lys Thr Thr Met Lys Ile Ile
1               5                   10                  15

Pro Phe Asn Arg Leu
                20

<210>  16

<211>  20

<212>  PRT

<213>  Artificial Sequence

<220>

<223> CαE - Cell penetrating peptidde

<400>  16

Arg Gly Ala Asp Tyr Ser Leu Arg Ala Val Arg Met Lys Ile Arg
1               5                   10                  15

Pro Leu Val Thr Gln
                20

<210>  17

<211>  24

<212>  PRT

<213> Homo sapiens

<220>
<223> hCT(9-32) - Cell penetrating peptide

<400>  17

Leu Gly Thr Tyr Thr Gln Asp Phe Asn Lys Phe His Thr Phe Pro
1               5                   10                  15

Gln Thr Ala Ile Gly Val Gly Ala Pro
                20

<210>  18

<211>  12

<212>  PRT

<213>  Artificial Sequence

<220>

<223>  HN-1 – Cell penetrating peptide

<400>  18

Thr Ser Pro Leu Asn Ile His Asn Gly Gln Lys Leu
1               5                   10

<210>  19

<211>  12

<212>  PRT

<213> Influenza virus

<220>
<223> Influenza virus nucleoprotein (NLS)-Cell penetrating peptide

<400>  19

Asn Ser Ala Ala Phe Glu Asp Leu Arg Val Leu Ser
1               5                   10

<210>  20

<211>  30

<212>  PRT

<213>  Artificial Sequence

<220>

<223>  KALA – Cell penetrating peptide

<400>  20

Trp Glu Ala Lys Leu Ala Lys Ala Leu Ala Lys Ala Leu Ala Lys
1               5                   10                  15

His Leu Ala Lys Ala Leu Ala Lys Ala Leu Lys Ala Cys Glu Ala
                20                  25                  30

<210>  21

<211>  10

<212>  PRT

<213>  Artificial Sequence

<220>

<223>  Ku70 – Cell penetrating peptide

<400>  21

Val Pro Met Leu Lys Pro Met Leu Lys Glu
1               5                   10

<210>  22

<211>  18

<212>  PRT

<213>  Artificial Sequence

<220>

<223>  MAP – Cell penetrating peptide

<400>  22

Lys Leu Ala Leu Lys Leu Ala Leu Lys Ala Leu Lys Ala Ala Leu Lys Leu
1               5                   10                  15

Ala

<210>  23

<211>  27

<212>  PRT

<213>  Artificial Sequence

<220>

<223>  MPG – Cell penetrating peptide

<400>  23

Gly Ala Leu Phe Leu Gly Phe Leu Gly Ala Ala Gly Ser Thr Met
1               5                   10                  15

Gly Ala Trp Ser Gln Pro Lys Lys Lys Arg Lys Val
                20                  25

<210>  24

<211>  16

<212>  PRT

<213>  Artificial Sequence

<220>

<223> MPM(IP/K-FGF) – Cell penetrating peptide

<400>  24

Ala Ala Val Ala Leu Leu Pro Ala Val Leu Leu Ala Leu Leu Ala Pro
1               5                   10                  15

<210>  25

<211>  9

<212>  PRT

<213>  Artificial Sequence

<220>

<223> N50(NLS of NF-κB P50) – Cell penetrating peptide

<400>  25

Val Gln Arg Lys Arg Gln Lys Leu Met
1               5

<210>  26

<211>  21

<212>  PRT

<213>  Artificial Sequence



<220>

<223> pep-1 – Cell penetrating peptide

<400>  26

Lys Glu Thr Trp Trp Glu Thr Trp Trp Thr Glu Trp Ser Gln Pro
1               5                   10                  15

Lys Lys Lys Arg Lys Val
                20



<210>  27

<211>  15

<212>  PRT

<213>  Artificial Sequence



<220>

<223> pep-7 – Cell penetrating peptide

<400>  27

Ser Asp Leu Trp Glu Met Met Met Val Ser Leu Ala Cys Gln Tyr
1               5                   10                  15


<210>  28

<211>  16

<212>  PRT

<213> Drosophila antennapedia



<220>

<223> Penetratin - Cell penetrating peptide

<400> 28

Arg Gln Ile Lys Ile Trp Phe Gln Asn Arg Arg Met Lys Trp Lys Lys
1               5                   10                  15

<210> 29

<211> 7

<212> PRT

<213> Drosophila antennapedia

<220>
<223> Short Penetratin - Cell penetrating peptide

<400> 29

Arg Arg Met Lys Trp Lys Lys
1               5

<210> 30

<211> 7

<212> PRT

<213> Artificial Sequence

<220>

<223> Poly Arginine-R7 - Cell penetrating peptide

<400> 30

Arg Arg Arg Arg Arg Arg Arg
1               5

<210> 31

<211> 9

<212> PRT

<213> Artificial Sequence

<220>

<223> Poly Arginine-R9 - Cell penetrating peptide

<400> 31

Arg Arg Arg Arg Arg Arg Arg Arg Arg
1               5


<210> 32

<211> 16

<212> PRT

<213> Rattus sp.



<220>
<223> pISL - Cell penetrating peptide

<400> 32

Arg Val Ile Arg Val Trp Phe Gln Asn Lys Arg Cys Lys Asp Lys Lys
1               5                   10                  15


<210> 33

<211> 28

<212> PRT

<213> Mus musculus



<220>
<223> Prion mouse PrPc1-28 - Cell penetrating peptide

<400> 33

Met Ala Asn Leu Gly Tyr Trp Leu Leu Ala Leu Phe Val Thr Met
1               5                   10                  15


Trp Thr Asp Val Gly Leu Cys Lys Lys Arg Pro Lys Pro
                20                  25


<210> 34

<211> 18

<212> PRT

<213> Mus musculus

<220>
<223> pVEC - Cell penetrating peptide

<400> 34

Leu Leu Ile Ile Leu Arg Arg Arg Ile Arg Lys Gln Ala His Ala His Ser
1               5                   10                  15

Lys

<210> 35

<211> 18

<212> PRT

<213> Artificial Sequence

<220>

<223> SAP - Cell penetrating peptide

<400> 35

Val Arg Leu Pro Pro Pro Val Arg Leu Pro Pro Pro Val Arg Leu Pro Pro
1               5                   10                  15

Pro

<210> 36

<211> 7

<212> PRT

<213> Simian virus 40

<220>
<223> SV-40 (NLS) - Cell penetrating peptide

<400> 36

Pro Lys Lys Lys Arg Lys Val
1               5

<210>  37

<211>  18

<212>  PRT

<213> Porcine

<220>
<223> SynB1 - Cell penetrating peptide

<400>  37

Arg Gly Gly Arg Leu Ser Tyr Ser Arg Arg Arg Phe Ser Thr Ser Thr Gly
1               5                   10                  15

Arg

<210>  38

<211>  10

<212>  PRT

<213> Porcine

<220>
<223> SynB3 - Cell penetrating peptide

<400>  38

Arg Arg Leu Ser Tyr Ser Arg Arg Arg Phe
1               5                   10

<210>  39

<211>  17

<212>  PRT

<213>  Artificial Sequence

<220>

<223>  SynB4 – Cell penetrating peptide

<400>  39

Ala Trp Ser Phe Arg Val Ser Tyr Arg Gly Ile Ser Tyr Arg Arg Ser Arg

1               5                   10                  15


<210>  40

<211>  14

<212>  PRT

<213> Human immunodeficiency virus type 1



<220>
<223> TAT47-60 - Cell penetrating peptide

<400>  40

Tyr Gly Arg Lys Lys Arg Arg Gln Arg Arg Arg Pro Pro Gln
1               5                   10


<210>  41

<211>  11

<212>  PRT

<213> Human immunodeficiency virus type 1



<220>
<223> TAT47-57 - Cell penetrating peptide

<400>  41

Tyr Gly Arg Lys Lys Arg Arg Gln Arg Arg Arg
1               5                   10


<210>  42

<211>  9

<212>  PRT

<213> Human immunodeficiency virus type 1

<220>
<223> TAT49-57 - Cell penetrating peptide

<400> 42

Arg Lys Lys Arg Arg Gln Arg Arg Arg
1               5


<210> 43

<211> 27

<212> PRT

<213> Artificial Sequence


<220>

<223> transportan - Cell penetrating peptide

<400> 43

Gly Trp Thr Leu Asn Ser Ala Gly Tyr Leu Leu Gly Lys Ile Asn
1               5                   10                  15

Leu Lys Ala Leu Ala Ala Leu Ala Lys Lys Ile Leu
                20                  25

<210> 44

<211> 21

<212> PRT

<213> Artificial Sequence


<220>

<223> transportan 10 - Cell penetrating peptide

<400> 44

Ala Gly Tyr Leu Leu Gly Lys Ile Asn Leu Lys Ala Leu Ala Ala
1               5                   10                  15

Leu Ala Lys Lys Ile Leu
                20

<210>  45

<211>  12

<212>  PRT

<213>  Artificial Sequence



<220>

<223> transportan derivative : - Cell penetrating peptide


<400>  45

Gly Trp Thr Leu Asn Ser Ala Gly Tyr Leu Leu Gly
1               5                   10


<210>  46

<211>  14

<212>  PRT

<213>  Artificial Sequence



<220>

<223> transportan derivative - Cell penetrating peptide


<400>  46

Ile Asn Leu Lys Ala Leu Ala Ala Leu Ala Lys Lys Ile Leu
1               5                   10


<210>  47

<211>  34

<212>  PRT

<213> herpes simplex virus 1



<220>
<223> VP22 - Cell penetrating peptide

<400>  47

Asp Ala Ala Thr Ala Thr Arg Gly Arg Ser Ala Ala Ser Arg Pro
1               5                   10                  15

Thr Glu Arg Pro Arg Ala Pro Ala Arg Ser Ala Ser Arg Pro Arg
                20                  25                  30

Arg Pro Val Asp

<210>  48

<211>  26

<212>  PRT

<213>  Artificial Sequence

<220>

<223>  VT5 - Cell penetrating peptide

<400>  48

Asp Pro Lys Gly Asp Pro Lys Gly Val Thr Val Thr Val Thr Val
1               5                   10                  15

Thr Val Thr Gly Lys Gly Asp Pro Lys Pro Asp
                20                  25

SEQUENCE LISTING

<110>  DIATOS S.A.

<120>  COMPOSITIONS AND METHODS FOR TREATING LYSOSOMAL STORAGE
DISEASES

<130>  46764/EP

<140>  EP 06290221.8
<141>  2006-02-08

<160>  48

<170>  PatentIn version 3.1

<210>  1

<211>  21

<212>  PRT

<213>  Artificial Sequence

<220>
<221>  Bufo gargarizans
<223>  Buforin II- Cell penetrating peptide

<400>  1

Thr Arg Ser Ser Arg Ala Gly Leu Gln Phe Pro Val Gly Arg Val
1               5                   10                  15

His Arg Leu Leu Arg Lys
                20

<210>  2

<211>  16

<212>  PRT

<213>  Artificial Sequence

<220>
<221> Homo sapiens
<223> DPV3-Cell penetrating peptide

<400>  2

Arg Lys Lys Arg Arg Arg Glu Ser Arg Lys Lys Arg Arg Arg Glu Ser
1               5                   10                  15

<210>  3

<211>  17

<212>  PRT

<213>  Artificial Sequence

<220>
<221> Homo sapiens
<223> DPV6-Cell penetrating peptide

<400>  3

Gly Arg Pro Arg Glu Ser Gly Lys Lys Arg Lys Arg Lys Arg Leu Lys Pro
1               5                   10                  15

<210>  4

<211>  15

<212>  PRT

<213>  Artificial Sequence

<220>
<221> Homo sapiens
<223> DPV7-Cell penetrating peptide

<400>  4

Gly Lys Arg Lys Lys Lys Gly Lys Leu Gly Lys Lys Arg Asp Pro
1               5                   10                  15

<210>  5

<211>  17

<212>  PRT

<213>  Artificial Sequence



<220>
<221> Homo sapiens
<223> DPV7b-Cell penetrating peptide

<400>  5

Gly Lys Arg Lys Lys Lys Gly Lys Leu Gly Lys Lys Arg Pro Arg Ser Arg
1               5                   10                  15


<210>  6

<211>  18

<212>  PRT

<213>  Artificial Sequence



<220>
<221> Homo sapiens
<223> DPV3/10-Cell penetrating peptide

<400>  6

Arg Lys Lys Arg Arg Arg Glu Ser Arg Arg Ala Arg Arg Ser Pro Arg His
1               5                   10                  15


Leu


<210>  7

<211>  19

<212>  PRT

<213>  Artificial Sequence



<220>

<221> Homo sapiens
<223> DPV10/6-Cell penetrating peptide

<400> 7

Ser Arg Arg Ala Arg Arg Ser Pro Arg Glu Ser Gly Lys Lys Arg Lys Arg
1               5                   10                  15

Lys Arg

<210> 8

<211> 19

<212> PRT

<213> Artificial Sequence

<220>
<221> Homo sapiens
<223> DPV1047-Cell penetrating peptide

<400> 8

Val Lys Arg Gly Leu Lys Leu Arg His Val Arg Pro Arg Val Thr Arg Met
1               5                   10                  15

Asp Val

<210> 9

<211> 18

<212> PRT

<213> Artificial Sequence

<220>
<221> Homo sapiens
<223> DPV1048-Cell penetrating peptide

<400> 9

Val Lys Arg Gly Leu Lys Leu Arg His Val Arg Pro Arg Val Thr Arg Asp
1               5                   10                  15

Val

<210>  10

<211>  14

<212>  PRT

<213>  Artificial Sequence

<220>
<221> Homo sapiens
<223> DPV10-Cell penetrating peptide

<400>  10

Ser Arg Arg Ala Arg Arg Ser Pro Arg His Leu Gly Ser Gly
1               5                   10

<210>  11

<211>  16

<212>  PRT

<213>  Artificial Sequence

<220>
<221> Homo sapiens
<223> DPV15-Cell penetrating peptide

<400>  11

Leu Arg Arg Glu Arg Gln Ser Arg Leu Arg Arg Glu Arg Gln Ser Arg
1               5                   10                  15

<210>  12

<211>  22

<212>  PRT

<213>  Artificial Sequence

<220>

<221> Homo sapiens
<223> DPV15b-Cell penetrating peptide

<400> 12

Gly Ala Tyr Asp Leu Arg Arg Arg Glu Arg Gln Ser Arg Leu Arg
1               5                   10                  15

Arg Arg Glu Arg Gln Ser Arg
                20

<210> 13

<211> 30

<212> PRT

<213> Artificial Sequence

<220>

<223> GALA - Cell penetrating peptide

<400> 13

Trp Glu Ala Ala Leu Ala Glu Ala Leu Ala Glu Ala Leu Ala Glu
1               5                   10                  15

His Leu Ala Glu Ala Leu Ala Glu Ala Leu Glu Ala Leu Ala Ala
                20                  25                  30

<210> 14

<211> 21

<212> PRT

<213> Artificial Sequence

<220>

<223> Cβ - Cell penetrating peptide

<400> 14

Lys Gly Ser Trp Tyr Ser Met Arg Lys Met Ser Met Lys Ile Arg
1               5                   10                  15

Pro Phe Phe Pro Gln Gln
                20

<210>  15

<211>  20

<212>  PRT

<213>  Artificial Sequence

<220>

<223> preCγ - Cell penetrating peptidde

<400>  15

Lys Thr Arg Tyr Tyr Ser Met Lys Lys Thr Thr Met Lys Ile Ile
1               5                   10                  15

Pro Phe Asn Arg Leu
                20

<210>  16

<211>  20

<212>  PRT

<213>  Artificial Sequence

<220>

<223> CαE - Cell penetrating peptidde

<400>  16

Arg Gly Ala Asp Tyr Ser Leu Arg Ala Val Arg Met Lys Ile Arg
1               5                   10                  15

Pro Leu Val Thr Gln
                20

<210>  17

<211>  24

<212>  PRT

<213>  Artificial Sequence

<220>
<221> Homo sapiens
<223> hCT(9-32) - Cell penetrating peptide

<400>  17

Leu Gly Thr Tyr Thr Gln Asp Phe Asn Lys Phe His Thr Phe Pro
1               5                   10                  15

Gln Thr Ala Ile Gly Val Gly Ala Pro
                20

<210>  18

<211>  12

<212>  PRT

<213>  Artificial Sequence

<220>

<223>  HN-1 - Cell penetrating peptide

<400>  18

Thr Ser Pro Leu Asn Ile His Asn Gly Gln Lys Leu
1               5                   10

<210>  19

<211>  12

<212>  PRT

<213>  Artificial Sequence

<220>
<221> Influenza virus
<223> Influenza virus nucleoprotein (NLS)-Cell penetrating peptide

<400>  19

Asn Ser Ala Ala Phe Glu Asp Leu Arg Val Leu Ser
1               5                   10


<210>  20

<211>  30

<212>  PRT

<213>  Artificial Sequence



<220>

<223>  KALA - Cell penetrating peptide

<400>  20

Trp Glu Ala Lys Leu Ala Lys Ala Leu Ala Lys Ala Leu Ala Lys
1               5                   10                  15


His Leu Ala Lys Ala Leu Ala Lys Ala Leu Lys Ala Cys Glu Ala
                20                  25                  30


<210>  21

<211>  10

<212>  PRT

<213>  Artificial Sequence



<220>

<223>  Ku70 - Cell penetrating peptide

<400>  21

Val Pro Met Leu Lys Pro Met Leu Lys Glu
1               5                   10


<210>  22

<211>  18

<212>  PRT

<213>  Artificial Sequence

<220>

<223>  MAP - Cell penetrating peptide

<400>  22

Lys Leu Ala Leu Lys Leu Ala Leu Lys Ala Leu Lys Ala Ala Leu Lys Leu
1               5                   10                  15

Ala

<210>  23

<211>  27

<212>  PRT

<213>  Artificial Sequence

<220>

<223>  MPG - Cell penetrating peptide

<400>  23

Gly Ala Leu Phe Leu Gly Phe Leu Gly Ala Ala Gly Ser Thr Met
1               5                   10                  15

Gly Ala Trp Ser Gln Pro Lys Lys Lys Arg Lys Val
                20                  25

<210>  24

<211>  16

<212>  PRT

<213>  Artificial Sequence

<220>

<223> MPM(IP/K-FGF) - Cell penetrating peptide

<400>  24

Ala Ala Val Ala Leu Leu Pro Ala Val Leu Leu Ala Leu Leu Ala Pro
1               5                   10                  15

<210>  25

<211>  9

<212>  PRT

<213>  Artificial Sequence

<220>

<223> N50(NLS of NF-κB P50) - Cell penetrating peptide

<400>  25

Val Gln Arg Lys Arg Gln Lys Leu Met
1               5

<210>  26

<211>  21

<212>  PRT

<213>  Artificial Sequence

<220>

<223> pep-1 - Cell penetrating peptide

<400>  26

Lys Glu Thr Trp Trp Glu Thr Trp Trp Thr Glu Trp Ser Gln Pro
1               5                   10                  15

Lys Lys Lys Arg Lys Val
                20

<210>  27

<211>  15

<212>  PRT

<213>  Artificial Sequence

<220>

<223> pep-7 - Cell penetrating peptide

<400>  27

Ser Asp Leu Trp Glu Met Met Met Val Ser Leu Ala Cys Gln Tyr
1               5                   10                  15

<210>  28

<211>  16

<212>  PRT

<213>  Artificial Sequence

<220>
<221> Drosophila antennapedia
<223> Penetratin - Cell penetrating peptide

<400>  28

Arg Gln Ile Lys Ile Trp Phe Gln Asn Arg Arg Met Lys Trp Lys Lys
1               5                   10                  15

<210>  29

<211>  7

<212>  PRT

<213>  Artificial Sequence

<220>
<221> Drosophila antennapedia
<223> Short Penetratin - Cell penetrating peptide

<400>  29

Arg Arg Met Lys Trp Lys Lys
1               5

<210>  30

<211>  7

<212>  PRT

<213>  Artificial Sequence

<220>

<223> Poly Arginine-R7 - Cell penetrating peptide

<400>  30

Arg Arg Arg Arg Arg Arg Arg
1               5

<210>  31

<211>  9

<212>  PRT

<213>  Artificial Sequence

<220>

<223> Poly Arginine-R9 - Cell penetrating peptide

<400>  31

Arg Arg Arg Arg Arg Arg Arg Arg Arg
1               5

<210>  32

<211>  16

<212>  PRT

<213>  Artificial Sequence

<220>
<221> Rattus sp.
<223> pISL - Cell penetrating peptide

<400>  32

Arg Val Ile Arg Val Trp Phe Gln Asn Lys Arg Cys Lys Asp Lys Lys
1               5                   10                  15

<210>  33

<211>  28

<212>  PRT

<213>  Artificial Sequence

<220>
<221> Mus musculus
<223> Prion mouse PrPc1-28 - Cell penetrating peptide

<400>  33

Met Ala Asn Leu Gly Tyr Trp Leu Leu Ala Leu Phe Val Thr Met
1               5                   10                  15

Trp Thr Asp Val Gly Leu Cys Lys Lys Arg Pro Lys Pro
                20                  25

<210>  34

<211>  18

<212>  PRT

<213>  Artificial Sequence

<220>
<221> Mus musculus
<223> pVEC - Cell penetrating peptide

<400>  34

Leu Leu Ile Ile Leu Arg Arg Arg Ile Arg Lys Gln Ala His Ala His Ser
1               5                   10                  15

Lys

<210>  35

<211>  18

<212>  PRT

<213>  Artificial Sequence

<220>

<223> SAP - Cell penetrating peptide

<400>  35

Val Arg Leu Pro Pro Pro Val Arg Leu Pro Pro Pro Val Arg Leu Pro Pro
1               5                   10                  15

Pro

<210>  36

<211>  7

<212>  PRT

<213>  Artificial Sequence

<220>
<221> Simian virus 40
<223> SV-40 (NLS) - Cell penetrating peptide

<400>  36

Pro Lys Lys Lys Arg Lys Val
1               5

<210>  37

<211>  18

<212>  PRT

<213>  Artificial Sequence

<220>
<221> Porcine
<223> SynB1 - Cell penetrating peptide

<400>  37

Arg Gly Gly Arg Leu Ser Tyr Ser Arg Arg Arg Phe Ser Thr Ser Thr Gly
1               5                   10                  15

Arg

<210>  38

<211>  10

<212>  PRT

<213>  Artificial Sequence

<220>
<221> Porcine
<223> SynB3 - Cell penetrating peptide

<400>  38

Arg Arg Leu Ser Tyr Ser Arg Arg Arg Phe
1               5                   10

<210>  39

<211>  17

<212>  PRT

<213>  Artificial Sequence

<220>

<223>  SynB4 - Cell penetrating peptide

<400>  39

Ala Trp Ser Phe Arg Val Ser Tyr Arg Gly Ile Ser Tyr Arg Arg Ser Arg

1               5                   10                  15

<210>  40

<211>  14

<212>  PRT

<213>  Artificial Sequence

<220>
<221> Human immunodeficiency virus type 1
<223> TAT47-60 - Cell penetrating peptide

<400>  40

Tyr Gly Arg Lys Lys Arg Arg Gln Arg Arg Arg Pro Pro Gln
1               5                   10

<210>  41

<211>  11

<212>  PRT

<213>  Artificial Sequence

<220>
<221> Human immunodeficiency virus type 1
<223> TAT47-57 - Cell penetrating peptide

<400>  41

Tyr Gly Arg Lys Lys Arg Arg Gln Arg Arg Arg
1               5                   10

<210>  42

<211>  9

<212>  PRT

<213>  Artificial Sequence

<220>
<221> Human immunodeficiency virus type 1
<223> TAT49-57 - Cell penetrating peptide

<400>  42

Arg Lys Lys Arg Arg Gln Arg Arg Arg
1               5

<210>  43

<211>  27

<212>  PRT

<213>  Artificial Sequence



<220>

<223> transportan - Cell penetrating peptide

<400>  43

Gly Trp Thr Leu Asn Ser Ala Gly Tyr Leu Leu Gly Lys Ile Asn
1               5                   10                  15


Leu Lys Ala Leu Ala Ala Leu Ala Lys Lys Ile Leu
                20                  25


<210>  44

<211>  21

<212>  PRT

<213>  Artificial Sequence



<220>

<223> transportan 10 - Cell penetrating peptide

<400>  44

Ala Gly Tyr Leu Leu Gly Lys Ile Asn Leu Lys Ala Leu Ala Ala
1               5                   10                  15


Leu Ala Lys Lys Ile Leu
                20


<210>  45

<211>  12

<212>  PRT

<213>  Artificial Sequence

<220>

<223> transportan derivative : - Cell penetrating peptide

<400> 45

Gly Trp Thr Leu Asn Ser Ala Gly Tyr Leu Leu Gly
1               5                   10

<210> 46

<211> 14

<212> PRT

<213> Artificial Sequence

<220>

<223> transportan derivative - Cell penetrating peptide

<400> 46

Ile Asn Leu Lys Ala Leu Ala Ala Leu Ala Lys Lys Ile Leu
1               5                   10

<210> 47

<211> 34

<212> PRT

<213> Artificial Sequence

<220>
<221> herpes simplex virus 1
<223> VP22 - Cell penetrating peptide

<400> 47

Asp Ala Ala Thr Ala Thr Arg Gly Arg Ser Ala Ala Ser Arg Pro
1               5                   10                  15

Thr Glu Arg Pro Arg Ala Pro Ala Arg Ser Ala Ser Arg Pro Arg
                20                  25                  30

Arg Pro Val Asp

<210> 48

<211> 26

<212> PRT

<213> Artificial Sequence

<220>

<223> VT5 - Cell penetrating peptide

<400> 48

Asp Pro Lys Gly Asp Pro Lys Gly Val Thr Val Thr Val Thr Val
1               5                   10                  15

Thr Val Thr Gly Lys Gly Asp Pro Lys Pro Asp
                20                  25

SEQUENCE LISTING

<110> DIATOS S.A.

<120> COMPOSITIONS AND METHODS FOR TREATING LYSOSOMAL STORAGE
DISEASES

<130> 46764/EP

<140> EP 06290221.8
<141> 2006-02-08

<160> 48

<170> PatentIn version 3.1

<210> 1

<211> 21

<212> PRT

<213> Artificial Sequence

<220>
<221> Bufo gargarizans
<223> Buforin II- Cell penetrating peptide

<400> 1

Thr Arg Ser Ser Arg Ala Gly Leu Gln Phe Pro Val Gly Arg Val
1               5                   10                  15

His Arg Leu Leu Arg Lys
                20

<210> 2

<211> 16

<212>  PRT

<213>  Artificial Sequence

<220>
<221> Homo sapiens
<223> DPV3-Cell penetrating peptide

<400>  2

Arg Lys Lys Arg Arg Arg Glu Ser Arg Lys Lys Arg Arg Arg Glu Ser
1               5                   10                  15

<210>  3

<211>  17

<212>  PRT

<213>  Artificial Sequence

<220>
<221> Homo sapiens
<223> DPV6-Cell penetrating peptide

<400>  3

Gly Arg Pro Arg Glu Ser Gly Lys Lys Arg Lys Arg Lys Arg Leu Lys Pro
1               5                   10                  15

<210>  4

<211>  15

<212>  PRT

<213>  Artificial Sequence

<220>
<221> Homo sapiens
<223> DPV7-Cell penetrating peptide

<400>  4

Gly Lys Arg Lys Lys Lys Gly Lys Leu Gly Lys Lys Arg Asp Pro
1               5                   10                  15

<210>  5

<211>  17

<212>  PRT

<213>  Artificial Sequence

<220>
<221> Homo sapiens
<223> DPV7b-Cell penetrating peptide

<400>  5

Gly Lys Arg Lys Lys Lys Gly Lys Leu Gly Lys Lys Arg Pro Arg Ser Arg
1               5                   10                  15

<210>  6

<211>  18

<212>  PRT

<213>  Artificial Sequence

<220>
<221> Homo sapiens
<223> DPV3/10-Cell penetrating peptide

<400>  6

Arg Lys Lys Arg Arg Arg Glu Ser Arg Arg Ala Arg Arg Ser Pro Arg His
1               5                   10                  15

Leu

<210>  7

<211>  19

<212>  PRT

<213>  Artificial Sequence

<220>

<221> Homo sapiens
<223> DPV10/6-Cell penetrating peptide

<400> 7

Ser Arg Arg Ala Arg Arg Ser Pro Arg Glu Ser Gly Lys Lys Arg Lys Arg
1               5                   10                  15

Lys Arg

<210> 8

<211> 19

<212> PRT

<213> Artificial Sequence

<220>
<221> Homo sapiens
<223> DPV1047-Cell penetrating peptide

<400> 8

Val Lys Arg Gly Leu Lys Leu Arg His Val Arg Pro Arg Val Thr Arg Met
1               5                   10                  15

Asp Val

<210> 9

<211> 18

<212> PRT

<213> Artificial Sequence

<220>
<221> Homo sapiens
<223> DPV1048-Cell penetrating peptide

<400> 9

Val Lys Arg Gly Leu Lys Leu Arg His Val Arg Pro Arg Val Thr Arg Asp
1               5                   10                  15

Val

<210> 10

<211> 14

<212> PRT

<213> Artificial Sequence

<220>
<221> Homo sapiens
<223> DPV10-Cell penetrating peptide

<400> 10

Ser Arg Arg Ala Arg Arg Ser Pro Arg His Leu Gly Ser Gly
1               5                   10

<210> 11

<211> 16

<212> PRT

<213> Artificial Sequence

<220>
<221> Homo sapiens
<223> DPV15-Cell penetrating peptide

<400> 11

Leu Arg Arg Glu Arg Gln Ser Arg Leu Arg Arg Glu Arg Gln Ser Arg
1               5                   10                  15

<210> 12

<211> 22

<212> PRT

<213> Artificial Sequence

<220>

<221> Homo sapiens
<223> DPV15b-Cell penetrating peptide

<400> 12

Gly Ala Tyr Asp Leu Arg Arg Arg Glu Arg Gln Ser Arg Leu Arg
1               5                   10                  15

Arg Arg Glu Arg Gln Ser Arg
                20

<210> 13

<211> 30

<212> PRT

<213> Artificial Sequence

<220>

<223> GALA - Cell penetrating peptide

<400> 13

Trp Glu Ala Ala Leu Ala Glu Ala Leu Ala Glu Ala Leu Ala Glu
1               5                   10                  15

His Leu Ala Glu Ala Leu Ala Glu Ala Leu Glu Ala Leu Ala Ala
                20                  25                  30

<210> 14

<211> 21

<212> PRT

<213> Artificial Sequence

<220>

<223> Cβ - Cell penetrating peptide

<400> 14

Lys Gly Ser Trp Tyr Ser Met Arg Lys Met Ser Met Lys Ile Arg
1               5                   10                  15

Pro Phe Phe Pro Gln Gln
                20

<210> 15

<211> 20

<212> PRT

<213> Artificial Sequence

<220>

<223> preCγ - Cell penetrating peptidde

<400> 15

Lys Thr Arg Tyr Tyr Ser Met Lys Lys Thr Thr Met Lys Ile Ile
1               5                   10                  15

Pro Phe Asn Arg Leu
                20

<210> 16

<211> 20

<212> PRT

<213> Artificial Sequence

<220>

<223> CαE - Cell penetrating peptidde

<400> 16

Arg Gly Ala Asp Tyr Ser Leu Arg Ala Val Arg Met Lys Ile Arg
1               5                   10                  15

Pro Leu Val Thr Gln
                20

<210> 17

<211>  24

<212>  PRT

<213>  Artificial Sequence

<220>
<221> Homo sapiens
<223> hCT(9-32) - Cell penetrating peptide

<400>  17

Leu Gly Thr Tyr Thr Gln Asp Phe Asn Lys Phe His Thr Phe Pro
1               5                   10                  15

Gln Thr Ala Ile Gly Val Gly Ala Pro
                20

<210>  18

<211>  12

<212>  PRT

<213>  Artificial Sequence

<220>

<223>  HN-1 - Cell penetrating peptide

<400>  18

Thr Ser Pro Leu Asn Ile His Asn Gly Gln Lys Leu
1               5                   10

<210>  19

<211>  12

<212>  PRT

<213>  Artificial Sequence

<220>
<221> Influenza virus
<223> Influenza virus nucleoprotein (NLS)-Cell penetrating peptide

<400> 19

Asn Ser Ala Ala Phe Glu Asp Leu Arg Val Leu Ser
1               5                   10

<210> 20

<211> 30

<212> PRT

<213> Artificial Sequence

<220>

<223> KALA - Cell penetrating peptide

<400> 20

Trp Glu Ala Lys Leu Ala Lys Ala Leu Ala Lys Ala Leu Ala Lys
1               5                   10                  15

His Leu Ala Lys Ala Leu Ala Lys Ala Leu Lys Ala Cys Glu Ala
                20                  25                  30

<210> 21

<211> 10

<212> PRT

<213> Artificial Sequence

<220>

<223> Ku70 - Cell penetrating peptide

<400> 21

Val Pro Met Leu Lys Pro Met Leu Lys Glu
1               5                   10

<210> 22

<211> 18

<212> PRT

<213>  Artificial Sequence

<220>

<223>  MAP - Cell penetrating peptide

<400>  22

Lys Leu Ala Leu Lys Leu Ala Leu Lys Ala Leu Lys Ala Ala Leu Lys Leu
1               5                   10                  15

Ala

<210>  23

<211>  27

<212>  PRT

<213>  Artificial Sequence

<220>

<223>  MPG - Cell penetrating peptide

<400>  23

Gly Ala Leu Phe Leu Gly Phe Leu Gly Ala Ala Gly Ser Thr Met
1               5                   10                  15

Gly Ala Trp Ser Gln Pro Lys Lys Lys Arg Lys Val
                20                  25

<210>  24

<211>  16

<212>  PRT

<213>  Artificial Sequence

<220>

<223> MPM(IP/K-FGF) - Cell penetrating peptide

<400>  24

Ala Ala Val Ala Leu Leu Pro Ala Val Leu Leu Ala Leu Leu Ala Pro
1               5                   10                  15

<210>  25

<211>  9

<212>  PRT

<213>  Artificial Sequence

<220>

<223> N50(NLS of NF-κB P50) - Cell penetrating peptide

<400>  25

Val Gln Arg Lys Arg Gln Lys Leu Met
1               5

<210>  26

<211>  21

<212>  PRT

<213>  Artificial Sequence

<220>

<223> pep-1 - Cell penetrating peptide

<400>  26

Lys Glu Thr Trp Trp Glu Thr Trp Trp Thr Glu Trp Ser Gln Pro
1               5                   10                  15

Lys Lys Lys Arg Lys Val
                20

<210>  27

<211>  15

<212>  PRT

<213>  Artificial Sequence

<220>

<223> pep-7 - Cell penetrating peptide

<400>  27

Ser Asp Leu Trp Glu Met Met Met Val Ser Leu Ala Cys Gln Tyr
1               5                   10                  15

<210>  28

<211>  16

<212>  PRT

<213>  Artificial Sequence

<220>
<221> Drosophila antennapedia
<223> Penetratin - Cell penetrating peptide

<400>  28

Arg Gln Ile Lys Ile Trp Phe Gln Asn Arg Arg Met Lys Trp Lys Lys
1               5                   10                  15

<210>  29

<211>  7

<212>  PRT

<213>  Artificial Sequence

<220>
<221> Drosophila antennapedia
<223> Short Penetratin - Cell penetrating peptide

<400>  29

Arg Arg Met Lys Trp Lys Lys
1               5

<210> 30

<211> 7

<212> PRT

<213> Artificial Sequence

<220>

<223> Poly Arginine-R7 - Cell penetrating peptide

<400> 30

Arg Arg Arg Arg Arg Arg Arg
1               5

<210> 31

<211> 9

<212> PRT

<213> Artificial Sequence

<220>

<223> Poly Arginine-R9 - Cell penetrating peptide

<400> 31

Arg Arg Arg Arg Arg Arg Arg Arg Arg
1               5

<210> 32

<211> 16

<212> PRT

<213> Artificial Sequence

<220>
<221> Rattus sp.
<223> pISL - Cell penetrating peptide

<400> 32

Arg Val Ile Arg Val Trp Phe Gln Asn Lys Arg Cys Lys Asp Lys Lys
1               5                   10                  15

<210>  33

<211>  28

<212>  PRT

<213>  Artificial Sequence

<220>
<221> Mus musculus
<223> Prion mouse PrPc1-28 - Cell penetrating peptide

<400>  33

Met Ala Asn Leu Gly Tyr Trp Leu Leu Ala Leu Phe Val Thr Met
1               5                   10                  15

Trp Thr Asp Val Gly Leu Cys Lys Lys Arg Pro Lys Pro
                20                  25

<210>  34

<211>  18

<212>  PRT

<213>  Artificial Sequence

<220>
<221> Mus musculus
<223> pVEC - Cell penetrating peptide

<400>  34

Leu Leu Ile Ile Leu Arg Arg Arg Ile Arg Lys Gln Ala His Ala His Ser
1               5                   10                  15

Lys

<210>  35

<211>  18

<212>  PRT

<213>  Artificial Sequence

<220>

<223> SAP - Cell penetrating peptide

<400>  35

Val Arg Leu Pro Pro Pro Val Arg Leu Pro Pro Pro Val Arg Leu Pro Pro
1               5                   10                  15

Pro

<210>  36

<211>  7

<212>  PRT

<213>  Artificial Sequence

<220>
<221> Simian virus 40
<223> SV-40 (NLS) - Cell penetrating peptide

<400>  36

Pro Lys Lys Lys Arg Lys Val
1               5

<210>  37

<211>  18

<212>  PRT

<213>  Artificial Sequence

<220>
<221> Porcine
<223> SynB1 - Cell penetrating peptide

<400>  37

Arg Gly Gly Arg Leu Ser Tyr Ser Arg Arg Arg Phe Ser Thr Ser Thr Gly
1               5                   10                  15

Arg

<210>  38

<211>  10

<212>  PRT

<213>  Artificial Sequence

<220>
<221> Porcine
<223> SynB3 - Cell penetrating peptide

<400>  38

Arg Arg Leu Ser Tyr Ser Arg Arg Arg Phe
1               5                   10

<210>  39

<211>  17

<212>  PRT

<213>  Artificial Sequence

<220>

<223>  SynB4 - Cell penetrating peptide

<400>  39

Ala Trp Ser Phe Arg Val Ser Tyr Arg Gly Ile Ser Tyr Arg Arg Ser Arg

1               5                   10                  15

<210>  40

<211>  14

<212>  PRT

<213>  Artificial Sequence

<220>
<221> Human immunodeficiency virus type 1
<223> TAT47-60 - Cell penetrating peptide

<400>  40

Tyr Gly Arg Lys Lys Arg Arg Gln Arg Arg Arg Pro Pro Gln
1               5                   10

<210>  41

<211>  11

<212>  PRT

<213>  Artificial Sequence

<220>
<221> Human immunodeficiency virus type 1
<223> TAT47-57 - Cell penetrating peptide

<400>  41

Tyr Gly Arg Lys Lys Arg Arg Gln Arg Arg Arg
1               5                   10

<210>  42

<211>  9

<212>  PRT

<213>  Artificial Sequence

<220>
<221> Human immunodeficiency virus type 1
<223> TAT49-57 - Cell penetrating peptide

<400>  42

Arg Lys Lys Arg Arg Gln Arg Arg Arg
1               5

<210>  43

<211>  27

<212>  PRT

<213>  Artificial Sequence



<220>

<223> transportan - Cell penetrating peptide

<400>  43

Gly Trp Thr Leu Asn Ser Ala Gly Tyr Leu Leu Gly Lys Ile Asn
1               5                   10                  15

Leu Lys Ala Leu Ala Ala Leu Ala Lys Lys Ile Leu
                20                  25

<210>  44

<211>  21

<212>  PRT

<213>  Artificial Sequence



<220>

<223> transportan 10 - Cell penetrating peptide

<400>  44

Ala Gly Tyr Leu Leu Gly Lys Ile Asn Leu Lys Ala Leu Ala Ala
1               5                   10                  15

Leu Ala Lys Lys Ile Leu
                20

<210>  45

<211>  12

<212>  PRT

<213>  Artificial Sequence

<220>

<223> transportan derivative : - Cell penetrating peptide

<400> 45

Gly Trp Thr Leu Asn Ser Ala Gly Tyr Leu Leu Gly
1               5                   10

<210> 46

<211> 14

<212> PRT

<213> Artificial Sequence

<220>

<223> transportan derivative - Cell penetrating peptide

<400> 46

Ile Asn Leu Lys Ala Leu Ala Ala Leu Ala Lys Lys Ile Leu
1               5                   10

<210> 47

<211> 34

<212> PRT

<213> Artificial Sequence

<220>
<221> herpes simplex virus 1
<223> VP22 - Cell penetrating peptide

<400> 47

Asp Ala Ala Thr Ala Thr Arg Gly Arg Ser Ala Ala Ser Arg Pro
1               5                   10                  15

Thr Glu Arg Pro Arg Ala Pro Ala Arg Ser Ala Ser Arg Pro Arg
                20                  25                  30

Arg Pro Val Asp

<210> 48

<211> 26

<212> PRT

<213> Artificial Sequence

<220>

<223> VT5 - Cell penetrating peptide

<400> 48

Asp Pro Lys Gly Asp Pro Lys Gly Val Thr Val Thr Val Thr Val
1               5                   10                  15

Thr Val Thr Gly Lys Gly Asp Pro Lys Pro Asp
                20                  25

SEQUENCE LISTING

<110>  DIATOS S.A.

<120>  COMPOSITIONS AND METHODS FOR TREATING LYSOSOMAL STORAGE DISEASES

<130>  DTS010

<160>  48

<170>  PatentIn version 3.1

<210>  1
<211>  21
<212>  PRT
<213>  Bufo gargarizans

<400>  1

Thr Arg Ser Ser Arg Ala Gly Leu Gln Phe Pro Val Gly Arg Val
1               5                   10                  15
His Arg Leu Leu Arg Lys
                20

<210>  2
<211>  16
<212>  PRT
<213>  Homo sapiens

<400>  2

Arg Lys Lys Arg Arg Arg Glu Ser Arg Lys Lys Arg Arg Arg Glu
1               5                   10                  15
Ser

<210>  3
<211>  17
<212>  PRT
<213>  Homo sapiens

<400>  3

Gly Arg Pro Arg Glu Ser Gly Lys Lys Arg Lys Arg Lys Arg Leu

1                   5                   10                  15

Lys Pro

<210>  4
<211>  15
<212>  PRT
<213>  Homo sapiens

<400>  4

Gly Lys Arg Lys Lys Lys Gly Lys Leu Gly Lys Lys Arg Asp Pro
1               5                   10                  15

<210>  5
<211>  17
<212>  PRT
<213>  Homo sapiens

<400>  5

Gly Lys Arg Lys Lys Lys Gly Lys Leu Gly Lys Lys Arg Pro Arg
1               5                   10                  15
Ser Arg

<210>  6
<211>  18
<212>  PRT
<213>  Homo sapiens

<400>  6

Arg Lys Lys Arg Arg Arg Glu Ser Arg Arg Ala Arg Arg Ser Pro
1               5                   10                  15
Arg His Leu

<210>  7
<211>  19
<212>  PRT
<213>  Homo sapiens

<400>  7

Ser Arg Arg Ala Arg Arg Ser Pro Arg Glu Ser Gly Lys Lys Arg
1               5                   10                  15
Lys Arg Lys Arg

<210>  8
<211>  19
<212>  PRT
<213>  Homo sapiens

<400>  8

Val Lys Arg Gly Leu Lys Leu Arg His Val Arg Pro Arg Val Thr
1               5                   10                  15
Arg Met Asp Val

<210>  9
<211>  18
<212>  PRT
<213>  Homo sapiens

<400>  9

Val Lys Arg Gly Leu Lys Leu Arg His Val Arg Pro Arg Val Thr
1               5                   10                  15
Arg Asp Val

<210>  10
<211>  14
<212>  PRT
<213>  Homo sapiens

<400>  10

Ser Arg Arg Ala Arg Arg Ser Pro Arg His Leu Gly Ser Gly
1               5                   10

<210>  11
<211>  16
<212>  PRT
<213>  Homo sapiens

<400>  11

Leu Arg Arg Glu Arg Gln Ser Arg Leu Arg Arg Glu Arg Gln Ser
1               5                   10                  15
Arg

<210>  12

<211>  22
<212>  PRT
<213>  Homo sapiens

<400>  12

Gly Ala Tyr Asp Leu Arg Arg Arg Glu Arg Gln Ser Arg Leu Arg
1               5                   10                  15
Arg Arg Glu Arg Gln Ser Arg
                20

<210>  13
<211>  30
<212>  PRT
<213>  Artificial Sequence

<220>
<223>  Artificial sequence

<400>  13

Trp Glu Ala Ala Leu Ala Glu Ala Leu Ala Glu Ala Leu Ala Glu
1               5                   10                  15
His Leu Ala Glu Ala Leu Ala Glu Ala Leu Glu Ala Leu Ala Ala
                20                  25                  30

<210>  14
<211>  21
<212>  PRT
<213>  unidentified

<400>  14

Lys Gly Ser Trp Tyr Ser Met Arg Lys Met Ser Met Lys Ile Arg
1               5                   10                  15
Pro Phe Phe Pro Gln Gln
                20

<210>  15
<211>  20
<212>  PRT
<213>  unidentified

<400>  15

Lys Thr Arg Tyr Tyr Ser Met Lys Lys Thr Thr Met Lys Ile Ile
1               5                   10                  15

Pro Phe Asn Arg Leu
                20

<210>  16
<211>  20
<212>  PRT
<213>  unidentified

<400>  16

Arg Gly Ala Asp Tyr Ser Leu Arg Ala Val Arg Met Lys Ile Arg
1               5                   10                  15
Pro Leu Val Thr Gln
                20

<210>  17
<211>  24
<212>  PRT
<213>  Homo sapiens

<400>  17

Leu Gly Thr Tyr Thr Gln Asp Phe Asn Lys Phe His Thr Phe Pro
1               5                   10                  15
Gln Thr Ala Ile Gly Val Gly Ala Pro
                20

<210>  18
<211>  12
<212>  PRT
<213>  Artificial Sequence

<220>
<223>  Artificial sequence
<400>  18

Thr Ser Pro Leu Asn Ile His Asn Gly Gln Lys Leu
1               5                   10

<210>  19
<211>  12
<212>  PRT
<213>  Influenza virus

<400>  19

Asn Ser Ala Ala Phe Glu Asp Leu Arg Val Leu Ser
1               5                   10

<210>  20
<211>  30
<212>  PRT

<213>  Artificial Sequence

<220>
<223>  Artificial sequence
<400>  20

Trp Glu Ala Lys Leu Ala Lys Ala Leu Ala Lys Ala Leu Ala Lys
1               5                   10                  15
His Leu Ala Lys Ala Leu Ala Lys Ala Leu Lys Ala Cys Glu Ala
                20                  25                  30

<210>  21
<211>  10
<212>  PRT
<213>  Artificial Sequence

<220>
<223>  Artificial sequence

<400>  21

Val Pro Met Leu Lys Pro Met Leu Lys Glu
1               5                   10

<210>  22
<211>  18
<212>  PRT
<213>  Artificial Sequence

<220>
<223>  Artificial sequence

<400>  22

Lys Leu Ala Leu Lys Leu Ala Leu Lys Ala Leu Lys Ala Ala Leu
1               5                   10                  15
Lys Leu Ala

<210>  23
<211>  27
<212>  PRT
<213>  Artificial Sequence

<220>
<223>  Artificial sequence

<400>  23

Gly Ala Leu Phe Leu Gly Phe Leu Gly Ala Ala Gly Ser Thr Met
1               5                   10                  15
Gly Ala Trp Ser Gln Pro Lys Lys Lys Arg Lys Val
                20                  25


<210>  24
<211>  16
<212>  PRT
<213>  Artificial Sequence


<220>
<223>  Artificial sequence
<400>  24

Ala Ala Val Ala Leu Leu Pro Ala Val Leu Leu Ala Leu Leu Ala
1               5                   10                  15
Pro


<210>  25
<211>  9
<212>  PRT
<213>  Artificial Sequence

<220>
<223>  Artificial sequence

<400>  25

Val Gln Arg Lys Arg Gln Lys Leu Met
1               5


<210>  26
<211>  21
<212>  PRT
<213>  Artificial Sequence


<220>
<223>  Artificial sequence

<400>  26

Lys Glu Thr Trp Trp Glu Thr Trp Trp Thr Glu Trp Ser Gln Pro
1               5                   10                  15
Lys Lys Lys Arg Lys Val

<210>  27
<211>  15
<212>  PRT
<213>  Artificial Sequence

<220>
<223>  Artificial sequence

<400>  27

Ser Asp Leu Trp Glu Met Met Met Val Ser Leu Ala Cys Gln Tyr
1               5                   10                  15

<210>  28
<211>  16
<212>  PRT
<213>  Drosophila antennapedia

<400>  28

Arg Gln Ile Lys Ile Trp Phe Gln Asn Arg Arg Met Lys Trp Lys
1               5                   10                  15
Lys

<210>  29
<211>  7
<212>  PRT
<213>  Drosophila antennapedia

<400>  29

Arg Arg Met Lys Trp Lys Lys
1               5

<210>  30
<211>  7
<212>  PRT
<213>  Artificial Sequence

<220>
<223>  Artificial sequence

<400>  30

Arg Arg Arg Arg Arg Arg Arg
1               5

<210>  31
<211>  9
<212>  PRT
<213>  Artificial Sequence

<220>
<223>  Artificial sequence

<400>  31

Arg Arg Arg Arg Arg Arg Arg Arg Arg
1               5

<210>  32
<211>  16
<212>  PRT
<213>  Rattus sp.

<400>  32

Arg Val Ile Arg Val Trp Phe Gln Asn Lys Arg Cys Lys Asp Lys
1               5                   10                  15
Lys

<210>  33
<211>  28
<212>  PRT
<213>  Artificial Sequence

<220>
<223>  Artificial sequence

<400>  33

Met Ala Asn Leu Gly Tyr Trp Leu Leu Ala Leu Phe Val Thr Met
1               5                   10                  15
Trp Thr Asp Val Gly Leu Cys Lys Lys Arg Pro Lys Pro
                20                  25

<210>  34
<211>  18
<212>  PRT
<213>  Mus musculus

<400>  34

Leu Leu Ile Ile Leu Arg Arg Arg Ile Arg Lys Gln Ala His Ala
1               5                   10                  15
His Ser Lys

<210>  35
<211>  18
<212>  PRT
<213>  Artificial Sequence

<220>
<223>  Artificial sequence

<400>  35

Val Arg Leu Pro Pro Pro Val Arg Leu Pro Pro Pro Val Arg Leu
1               5                   10                  15
Pro Pro Pro

<210>  36
<211>  7
<212>  PRT
<213>  Simian virus 40

<400>  36

Pro Lys Lys Lys Arg Lys Val
1               5

<210>  37
<211>  18
<212>  PRT
<213>  Porcine leukocyte

<400>  37

Arg Gly Gly Arg Leu Ser Tyr Ser Arg Arg Arg Phe Ser Thr Ser
1               5                   10                  15
Thr Gly Arg

<210>  38
<211>  10
<212>  PRT
<213>  Porcine leukocyte

<400>  38

Arg Arg Leu Ser Tyr Ser Arg Arg Arg Phe

1                   5                       10

<210>  39
<211>  17
<212>  PRT
<213>  Unidentified

<400>  39

Ala Trp Ser Phe Arg Val Ser Tyr Arg Gly Ile Ser Tyr Arg Arg
1               5                   10                  15
Ser Arg

<210>  40
<211>  14
<212>  PRT
<213>  Human immunodeficiency virus type 1

<400>  40

Tyr Gly Arg Lys Lys Arg Arg Gln Arg Arg Arg Pro Pro Gln
1               5                   10

<210>  41
<211>  11
<212>  PRT
<213>  Human immunodeficiency virus type 1

<400>  41

Tyr Gly Arg Lys Lys Arg Arg Gln Arg Arg Arg
1               5                   10

<210>  42
<211>  9
<212>  PRT
<213>  Human immunodeficiency virus type 1

<400>  42

Arg Lys Lys Arg Arg Gln Arg Arg Arg
1               5

<210>  43
<211>  27
<212>  PRT

<213>  Artificial Sequence

<220>
<223>  Artificial sequence

<400>  43

Gly Trp Thr Leu Asn Ser Ala Gly Tyr Leu Leu Gly Lys Ile Asn
1               5                   10                  15

Leu Lys Ala Leu Ala Ala Leu Ala Lys Lys Ile Leu
                20                  25

<210>  44
<211>  21
<212>  PRT
<213>  Artificial Sequence

<220>
<223>  Artificial sequence
<400>  44

Ala Gly Tyr Leu Leu Gly Lys Ile Asn Leu Lys Ala Leu Ala Ala
1               5                   10                  15
Leu Ala Lys Lys Ile Leu
                20

<210>  45
<211>  12
<212>  PRT
<213>  Artificial Sequence

<220>
<223>  Artificial sequence
<400>  45

Gly Trp Thr Leu Asn Ser Ala Gly Tyr Leu Leu Gly
1               5                   10

<210>  46
<211>  14
<212>  PRT
<213>  Artificial Sequence

<220>
<223>  Artificial sequence

<400>  46

Ile Asn Leu Lys Ala Leu Ala Ala Leu Ala Lys Lys Ile Leu
1               5                   10

<210>  47
<211>  34
<212>  PRT
<213>  herpes simplex virus 1

<400>  47

Asp Ala Ala Thr Ala Thr Arg Gly Arg Ser Ala Ala Ser Arg Pro
1               5                   10                  15
Thr Glu Arg Pro Arg Ala Pro Ala Arg Ser Ala Ser Arg Pro Arg
                20                  25                  30
Arg Pro Val Asp

<210>  48
<211>  26
<212>  PRT
<213>  Artificial Sequence

<220>
<223>  Artificial sequence

<400>  48

Asp Pro Lys Gly Asp Pro Lys Gly Val Thr Val Thr Val Thr Val
1               5                   10                  15
Thr Val Thr Gly Lys Gly Asp Pro Lys Pro Asp
                20                  25

## Description

### FIELD OF THE INVENTION

The invention relates generally to compositions and methods of treating lysosomal storage disorders (LSDs) and more particularly to the use of cell-penetrating peptides (CPPs) for enhancing the efficacy of enzyme replacement therapy for LSDs.

### BACKGROUND OF THE INVENTION

Enzyme replacement therapy ("ERT"), which consists of an exogenous supply of a missing enzyme, has been the most successful therapeutic approach for certain lysosomal storage disorders. This therapy relies on the ability of the cells to take up lysosomal enzymes through a mannose-6-phosphate receptor-mediated endocytosis pathway.

In mammalian cells, two types of mannose 6-phosphate receptors ("M6PR") have been described: a cation-independant MPR (CI-MPR) of about 270 kDa, also known as the insulin-like growth factor II receptor (IGF-IIR), and the 46 kDa cation-dependant MPR (CD-MPR). Both are type I glycoproteins that have a high binding affinity for lysosomal enzymes and other proteins that contain phosphomannosyl residues. The CI- and CD-MPR are found predominantly inside the cell where they play key roles in targeting mannose-6-phosphate ("M6P")-expressing proteins from the Golgi to a pre-lysosomal compartment (Griffiths et al., J. Cell Sci. 95:441-461 (1990)). Both receptors are also present at the cell surface, although the IGF-II/CI-MP receptor is thought to be the predominant receptor for the binding and uptake of M6P-containing molecules (Stein et al., EMBO J. 6:2677-2681 (1987); Ma et al., J. Biol. Chem. 266:10589-10595 (1991)). The IGF-II/CI-MP receptor is ubiquitously expressed in cells and tissues, but a number of studies have demonstrated that the expression level of this receptor is both tissue-specific and developmentally regulated manner (Hawkes and Kar, Brain Res. Rev. 44:117-140 (2004)).

Lysosomal storage disorders are a group of more than 40 heritable diseases that are usually caused by a deficiency of a specific single critical enzyme. This loss in enzymatic activity results in the progressive accumulation of undegradated substrate such as sphingolipids, glycogen, mucopolysaccharides or glycoproteins within the lysosomes with resultant engorgement of the organelle. This leads to cellular and tissue damage, subsequent organ dysfunction and in some diseases to early mortality. Based on ligand binding properties of the IGF-II/CI-MP receptor, recent clinical trials have demonstrated that enzyme replacement therapy with recombinant enzyme constitutes a major clinical advance in the treatment of patients with such lysosomal storage diseases (as Fabry or Gaucher diseases) (Desnick and Schuchman, Nat. Rev. Genet. 3:954-966 (2002), Erratum in: Nat Rev Genet. 4:157 (2003); Sly, Mo. Med. 101:100-104 (2004)).

Nevertheless, efficient therapy requires the use of high doses of M6P-glycosylated recombinant proteins to allow sufficient internalization and targeting of M6P-glycosylated proteins. M6PR expression is low in skeletal muscles, a major target tissue for ERT, and an age-related loss of transport system mediated by M6PR across the blood brain barrier is observed, which is a problem for treatment and particularly for the neurological signs observed in some LSDs (Funk et al., J. Clin. Endocrinol. Metab. 75:424-431 (1992); Raben et al., Mol. Genet. Metab. 80:159-169 (2003); Urayama et al., Proc. Natl. Acad. Sci. U S A. 101:12658-12663 (2004); Wenk et al., Biochem. Int. 23:723-731 (1991)). In addition, the recombinant proteins used for ERT must be synthesized by expensive mammalian systems, which sometime poorly modify recombinant enzymes with M6P (Zhu et al., J. Biol. Chem. 279:50336-50341 (2004)).

Further, it was showed that the recombinant acid alpha-glucosidase produced in CHO cells needed to be remodeled by direct chemical conjugation of M6P-carbohydrate moieties to enhance its delivery to the affected muscles in Pompe disease animal model (Zhu et al., Biochem. J. 20: (2005)).

Using the capacity of IGF-II/CI-MPR to bind IGF-II with high affinity on distinct site of M6P tag, LeBowitz et al. (Proc. Natl. Acad. Sci. USA. 101:3083-3088 (2004)) designed a polypeptide sequence from human IGF-II (60 amino acids) named "GILT tag" to circumvent glycosylation-dependant targeting. GILT tag sequence was fused to beta-glucuronidase gene and allowed delivery of the recombinant enzyme without loss of the endocytosis mediated IGF-II/CI-MP receptor internalization (see also patent applications WO 02/087510 and WO 03/102583).

PCT patent application WO 02/055684 and Xia et al. (Nat. Biotechnol. 19:640-644 (2001)) disclose a recombinant protein transduction domain (PTD)-fusion protein comprising a lysosomal enzyme linked to the Tat protein transduction domain (Tat₄₇₋₅₇ and Tat₅₇₋₄₇). The authors showed *in vitro* that beta-glucuronidase modified at the COOH terminus with the PTD of Tat allowed for both M6P dependant and independent entry (compared to total inhibition of uptake of native beta-glucuronidase by M6P). However the ability of the Tat peptide to deliver the protein was evaluated *in vivo* by using recombinant viral vectors, which were injected in mice.

PCT patent application WO 04/108071 relates to a chimeric Central Nervous System (CNS) targeting polypeptide, comprising a payload polypeptide domain comprising such as a lysosomal enzyme (e. g., beta-glucuronidase) and a Blood Brain Barrier (BBB)-receptor binding domain from Apolipoprotein B, Apolipoprotein E or insulin-like growth factor for example. However, the BBB receptor binding domain is not considered as being a cell-penetrating peptide. Orii et al. (Mol. Ther. 12:345-352 (2005)) used recombinant forms of human beta-glucuronidase purified from secretions from stably transfected CHO cells to compare the native (phosphomannosylated) enzyme to a beta-glucuronidase-Tat C-terminal fusion protein containing the 11 amino acid HIV Tat protein transduction domain. Produced recombinant beta-glucuronidase-Tat fusion protein was less (46%) phosphorylated than the native enzyme. The authors found that the beta-glucuronidase-Tat fusion protein showed about 50% more uptake than the native beta-glucuronidase uptake by cultured human fibroblasts. They also showed that the native beta-glucuronidase uptake is exclusively mediated by the M6P receptor but that beta-glucuronidase-Tat fusion protein showed only 30-50% as much M6P receptor-mediated uptake, and was taken up by adsorptive endocytosis through binding of the positively charged Tat peptide to cell surface proteoglycans.

However, a need for a method for improving the lysosomal enzyme replacement therapy, *i. e*., the delivery of lysosomal enzymes into the cells, still exists.

### SUMMARY OF THE INVENTION

The invention relates to chimeric polypeptides comprising a lysosomal peptide fused or conjugated to at least one cell-penetrating peptide (CPP). Also provided by the invention are methods for treating a subject suffering from lysosomal storage disorders (LSD).

In one aspect, the invention provides chimeric polypeptides having at least two domains fused or conjugated together: the first domain comprises a cell-penetrating peptide which facilitates active transport across a biological membrane and into lysosomes, and the second domain comprises a lysosomal enzyme.

The compositions of the invention are based in part on the discovery that certain CPPs allow for transport molecules across biological membranes into lysosomes.

Preferably, the CPP comprises at least four basic amino acids and / or binds a glycosaminoglycan such as heparin, heparin sulphate or chondroitin sulfate.

Advantageously, the invention also permits targeting of the therapeutic compound such as a lysosomal enzyme (*e. g*., beta-glucuronidase) to a lysosome in a mannose-6-phospate (M6P)-independent manner.

The invention also provides a polynucleotide (DNA or RNA) encoding a lysosomal enzyme fused to a nucleotide sequence encoding a cell-penetrating peptide. Nucleotide sequence encoding CPPs can be determined by those skilled in the art based on the information contained within the genetic code.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. All publications, patent applications, patents and other references mentioned herein are incorporated by reference in their entirety. In the case of conflict, the present Specification, including definitions, will control.

### DETAILED DESCRIPTION OF THE INVENTION

The invention provides chimeric polypeptides comprising a lysosomal enzyme fused or conjugated to at least one cell-penetrating peptide. The chimeric polypeptides of the invention or nucleic acids encoding these chimeric polypeptides can be incorporated into a composition, preferably pharmaceutical composition, and administered to a subject suffering from lysosomal storage disorders (LSD). Accordingly, the invention provides a chimeric polypeptide comprising at least two polypeptide domains which can occur in any order in the chimeric polypeptide, and the chimeric polypeptide can include one or more of each domain.

As used herein, "chimeric polypeptide(s)" comprise(s) at least a lysosomal enzyme coupled to a non-lysosomal peptide (*e. g*., a CPP).

The term "coupled" indicates that the lysosomal enzyme is "fused" or "conjugated" to a non-lysosomal peptide, such as a CPP. The CPP can be coupled (fused or conjugated) to the N-terminus or C-terminus of the lysosomal enzyme.

The term "fused" indicates that the chimeric polypeptide of the invention can be synthesized as a fusion protein comprising both domains (i. e., the lysosomal enzyme and the CPP). When used to refer to nucleic acids encoding a chimeric polypeptide, the term "fused" means that a nucleic acid encoding the first and the second domain are fused in-frame to each other by genetic engineering. The term "conjugated" is intended to indicate that the first and second domains of the chimeric polypeptide (*i. e.*, the lysosomal enzyme and the CPP) are chemically linked; most typically via a covalent bond such as a peptide bond in a manner that allows for at least one function associated with the lysosomal enzyme. The conjugation can be accomplished via a cross-linking reagent.

As used herein, the term "lysosomal enzyme" shall be interpreted to encompass naturally extracted or recombinantly produced lysosomal enzyme that is biologically active in an organelle called the lysosome and associated with a Lysosomal Storage Disorder (LSD) Lysosomal enzymes degrade (*i.e*. break down) macromolecules (*i.e*. large molecules) and other materials (such as bacteria) that have been taken up by the cell during the process of endocytosis. By "biologically active" is meant the enzyme has the ability to suppress, prevent the symptoms of a Lysosomal Storage Disorder (LSD) or rescue a LSD gene defect. Biologically active lysosomal enzymes are selected from the group comprising Acid-alpha-1, 4-glucosidase; beta-Galactosidase; beta-Galactosidase A; beta-Hexosaminidase A; beta-Hexosaminidase B; GM₂ Activator Protein; Glucocerebrosidase; Arylsulfatase A; Arylsulfatase B; Galactosylceramidase; Acid Sphigomyelinase; Cholesterol; Acid Ceramidase; Acid Lipase; alpha-L-Iduronidase; Iduronidase Sulfatase; Heparan N-Sulfatase; alpha-N-Acetylglucosaminidase; Acetyl-CoA-Glucosaminide Acetyltransferase; N-Acetylglucosamine-6-Sulfatase; Galactosamine-6-Sulfatase; beta-Glucuronidase; alpha-Mannosidase; beta-Mannosidase; alpha-L-Fucosidase; N-Aspartyl-beta-Glucosaminidase; alpha-Neuraminidase; Lysosomal Protective Protein; alpha-N-Acetyl-Galactosaminidase; N-Acetylglucosamine-1-Phosphotransferase; Cystine Transport Protein; Sialic Acid Transport Protein; Palmitoyl-Protein Thioesterase; Saposins A, B, C or D; Cathepsin protein family. Lysosomal enzymes can be derived from any species such as human, bovine, porcine, equine, canine or murine.

One particular preferred lysosomal enzyme is beta-Glucuronidase. The molecular weight of the human non-phosphomanosylated beta-glucuronidase is about 70 kDa per subunit.

The term "lysosomal enzyme derivative" is intended to encompass any form of a native (or naturally occurring) lysosomal enzyme as defined above, wherein one or more of the amino acids within the polypeptide chain has been replaced with an alternative amino acid and/or wherein one or more of the amino acids has been deleted or wherein one or more additional amino acids has been added to the polypeptide chain or amino acid sequences of said lysosomal enzyme, which still has at least one function of the native lysosomal enzyme, *i.e.* which is still biologically active. A lysosomal enzyme derivative exhibit at least about 50%, preferably at least about 70%, more preferably at least about 80%-85%, preferably at least about 90% and most preferably at least about 95%-99% amino acid sequence homology over a defined native lysosomal enzyme. Many suitable computer programs for calculating the "homology" between two amino acid sequences are generally known in the art, such as BLAST program (http://www.ncbi.nlm.nih.gov/BLAST/) choosing the scoring matrix BLOSUM62.

The term "lysosomal enzyme" and "lysosomal enzyme derivative" are used interchangeably herein.

The lysosomal enzymes can be used phosphomannosylated (i. e., phosphorylated) or non phosphomannosylated (*i.e.,* non- phosporylated). During lysosomal enzymes biosynthesis, enzymes are glycosylated in the endoplasmic reticulum and phosphorylated on high mannose glycans in Golgi. Phosphomannosyl units acquisition allows binding of the enzymes to M6PR and subsequent translocation to lysosome. According to a preferred embodiment the lysosomal enzyme is used non phosphomannosylated.

Lysosomal enzymes for use in the present invention can be obtained from numerous commercial sources such as Sigma-Aldrich, Prozyme.

The lysosomal polypeptides or nucleic acids encoding a lysosomal enzyme or derivative thereof can be constructed using any lysosomal enzyme amino acid sequences or encoding sequences known in the art. Sources for such sequences include UniProtKB/Swiss-Prot Database. For example, it is given the Swiss-Prot accession number of certain homo sapiens enzymes: Beta-glucuronidase P08236; Alpha-L-iduronidase P35475; Glucosylceramidase/Acid beta-glucosidase P04062; Acid sphingomyelinase P17405; Alpha-galactosidase A P06280; Galactocerebrosidase P54803; Sialidase 1 Q99519; Lysosomal alpha-mannosidase 000754; Beta-mannosidase U60337; and Cathepsin K P43235.

"Lysosomal Storage Disorders" (LSDs) comprise but are not limited to the Glycogenosis Disorders such as Pompe Disease; the Glycolipidosis Disorders such as GM1 Gangliosidosis, GM2 Gangliosidosis AB Variant; Tray-Sachs Disease, Sanfhoff Disease, Fabry Disease, Gaucher Disease, Metachromatic Leukodystrophy, Krabbe Disease, Nieman-Pick Types A, B or C, Farber Disease, Wolman Disease; the Mucopolysaccharide Disorders such as Hurler Syndrome (MPS IH), Scheie Syndrome (MPS IS), Hurler-Scheie Syndrome (MPS IH/S), Hunter Syndrome (MPS II), Sanfilippo A (MPS IIIA), B (MPS IIIB), C (MPS IIIC) or D (MPS IIID) Syndrome, Morquio A (MPS IVA) or B (MPS IVB) Syndrome, Maroteaux-Lamy Syndrome (MPS VI), Sly Syndrom (MPS VII); the Oligosaccharide/Glycoprotein Disorders such as alpha-Manosidosis, beta-Mannosidosis, Fucosidosis, Asparylglucosaminuria, Sialidosis (Mucolipidosis I), Galactosialidosis (Goldberg Syndrome), Schindler Disease; the Lysosomal Enzyme Transport Disorders such as Mucolipidosis II (I-Cell Disease), Muculipidosis III (Pseudo-Hurler Polydystrophy); the Lysosomal Membrane Transport Disorders such as Cystinosis, Salla Disease, Infantile Sialic Acid Storage Disease; or Batten Disease (Juvenil Neuronal Ceroid Lipofuscinosis), Infantile Neuronal Ceroid Lipofuscinosis, Mucolipidosis IV, Prosaposin.

The term "cell penetrating peptide(s)" (CPP(s)) is defined as a carrier peptide that is capable of crossing biological membrane or a physiological barrier. Cell penetrating peptides are also called cell-permeable peptides, protein-transduction domains (PTD) or membrane-translocation sequences (MTS). CPPs have the ability to translocate *in vitro* and/or *in vivo* the mammalian cell membranes and enter into cells and/or cell nuclei, and directs a conjugated compound of interest, such as a drug or marker, to a desired cellular destination. Accordingly, the CPP can direct or facilitate penetration of a compound of interest across a phospholipid, mitochondrial, endosomal or nuclear membrane. The CPP can also direct a compound of interest from outside the cell through the plasma membrane, and into the cytoplasm or to a desired location within the cell, *e.g*., a lysosome, the nucleus, the ribosome, the mitochondria, the endoplasmic reticulum, or a peroxisome. Alternatively or in addition, the CPP can direct a compound of interest across the blood-brain, trans-mucosal, hematoencephalic, hematoretinal, skin, gastrointestinal and/or pulmonary barriers.

Penetration across a biological membrane or a physiological barrier can be determined by various processes, for example by a cell penetration test having a first incubation step for the CPP conjugated to a marker in the presence of culture cells, followed by a fixating step, and then revelation of the presence of the peptide inside the cell. In another embodiment, the revelation step can be done with an incubation of the CPP in the presence of labeled antibodies and directed against the CPP, followed by detection in the cytoplasm or in immediate proximity of the cell nucleus, or even within it, of the immunologic reaction between the CPP's amino acid sequence and the labeled antibodies. Revelation can also be done by marking an amino acid sequence in the CPP and detecting the presence of the marking in the cell compartments. Cell penetration tests are well known to those skilled in the art. However, for example a cell penetration test was described in the above-mentioned patent application No WO 97/02840.

Several proteins and their peptide derivatives have been found to possess cell internalization properties including but not limited to the Human Immunodeficency Virus type 1 (HIV-1) protein Tat (Ruben et al. J. Virol. 63, 1-8 (1989)), the herpes virus tegument protein VP22 (Elliott and O'Hare, Cell 88, 223-233 (1997)), the homeotic protein of *Drosophila melanogaster* Antennapedia (the CPP is called Penetratin) (Derossi et al., J. Biol. Chem. 271, 18188-18193 (1996)), the protegrin 1 (PG-1) anti-microbial peptide SynB (Kokryakov et al., FEBS Lett. 327, 231-236 (1993)) and the basic fibroblast growth factor (Jans, Faseb J. 8, 841-847 (1994)). A number of other proteins and their peptide derivatives have been found to possess similar cell internalization properties. The carrier peptides that have been derived from these proteins show little sequence homology with each other, but are all highly cationic and arginine or lysine rich. Indeed, synthetic poly-arginine peptides have been shown to be internalized with a high level of efficiency (Futaki et al., J. Mol. Recognit. 16, 260-264 (2003); Suzuki et al., J. Biol. Chem. (2001)).

CPP can be of any length. For example CPP is less than or equal to 500, 250, 150, 100, 50, 25, 10, 6 or 4 amino acids in length. For example CPP is greater than or equal to 4, 6, 10, 25, 50, 100, 150, 250 or 500 amino acids in length. The suitable length and design of the CPP will be easily determined by those skilled in the art. As general references on CPPs it can be cited: CELL PENETRATING PEPTIDES: PROCESSES AND APPLICATIONS, edited by Ulo Langel (2002); or Advanced Drug Delivery Reviews 57:489-660 (2005).

In preferred embodiments the CPP is 6, 7, 5, 6, 7, 8, 9, 10, 11, 12, 13, 14, 15, 16, 17, 18, 19, 20, 22, 23, 24 or 25 amino acids in length.

The cell penetrating peptides according to the invention can be, but not limited to, those described below or variants thereof. A "variant" that is at least about 50%, preferably at least about 70%, more preferably at least about 80%-85%, preferably at least about 90% and most preferably at least about 95%-99% identical thereto. For example, peptides can have substitutions at 1, 2, 3, 4 or more residues. The CPP can be used in their natural form (such as described above) or polymer form (dimer, trimer, etc.).

**Table 1: Selection of well-known cell penetrating peptide used for cargo delivery**

| SEQ ID NO: | Cell Penetrating Peptides | Amino acid sequences (Nter to Cter) in one letter code |
|---|---|---|
| 1 | Buforin II | TRSSRAGLQFPVGRVHRLLRK |
| 2 | DPV3 | RKKRRRESRKKRRRES |
| 3 | DPV6 | GRPRESGKKRKRKRLKP |
| 4 | DPV7 | GKRKKKGKLGKKRDP |
| 5 | DPV7b | GKRKKKGKLGKKRPRSR |
| 6 | DPV3/10 | RKKRRRESRRARRSPRHL |
| 7 | DPV10/6 | SRRARRSPRESGKKRKRKR |
| 8 | DPV1047 | VKRGLKLRHVRPRVTRMDV |
| 9 | DPV1048 | VKRGLKLRHVRPRVTRDV |
| 10 | DPV10 | SRRARRSPRHLGSG |
| 11 | DPV 15 | LRRERQSRLRRERQSR |
| 12 | DPV 15b | GAYDLRRRERQSRLRRRERQSR |
| 13 | GALA | WEAALAEALAEALAEHLAEALAEALEALAA |
| | Haptotactic peptides | |
| 14 | Cβ | KGSWYSMRKMSMKIRPFFPQQ |
| 15 | preCγ | KTRYYSMKKTTMKIIPFNRL |
| 16 | CαE | RGADYSLRAVRMKIRPLVTQ |
| 17 | hCT(9-32) | LGTYTQDFNKFHTFPQTAIGVGAP |
| 18 | HN-1 | TSPLNIHNGQKL |
| 19 | Influenza virus nucleoprotein (NLS) | NSAAFEDLRVLS |
| 20 | KALA | WEAKLAKALAKALAKHLAKALAKALKACEA |
| 21 | Ku70 | VPMLKPMLKE |
| 22 | MAP | KLALKLALKALKAALKLA |
| 23 | MPG | GALFLGFLGAAGSTMGAWSQPKKKRKV |
| 24 | MPM (IP/K-FGF) | AAVALLPAVLLALLAP |
| 25 | N50 (NLS ofNF-κB P50) | VQRKRQKLM |
| 26 | Pep-1 | KETWWETWWTEWSQPKKKRKV |
| 27 | Pep-7 | SDLWEMIVIIVIVSLACQY |
| 28 | Penetratin | RQIKIWFQNRRMKWKK |
| 29 | Short Penetratin | RRMKWKK |
| 30 | Poly Arginine - R₇ | RRRRRRR |
| 31 | Poly Arginine - R₉ | RRRRRRRRR |
| 32 | pISL | RVIRVWFQNKRCKDKK |
| 33 | Prion mouse PrPc₁₋₂₈ | MANLGYWLLALFVTMWTDVGLCKKRPKP |
| 34 | pVEC | LLIILRRRIRKQAHAHSK |
| 35 | SAP | VRLPPPVRLPPPVRLPPP |
| 36 | SV-40 (NLS) | PKKKRKV |
| 37 | SynB1 | RGGRLSYSRRRFSTSTGR |
| 38 | SynB3 | RRLSYSRRRF |
| 39 | SynB4 | AWSFRVSYRGISYRRSR |
| 40 | Tat₄₇₋₆₀ | YGRKKRRQRRRPPQ |
| 41 | Tat₄₇₋₅₇ | YGRKKRRQRRR |
| 42 | Tat₄₉₋₅₇ | RKKRRQRRR |
| 43 | Transportan | GWTLNSAGYLLGKINLKALAALAKKIL |
| 44 | Transportan 10 | AGYLLGKINLKALAALAKKIL |
| 45 | Transportan derivative: | GWTLNSAGYLLG |
| 46 | Transportan derivative | INLKALAALAKKIL |
| 47 | VP22 | DAATATRGRSAASRPTERPRAPARSASRPRRPVD |
| 48 | VT5 | DPKGDPKGVTVTVTVTVTGKGDPKPD |

If necessary, several well known chemical strategies can be used by one skilled in the art for transforming a CPP into a drug candidate with increased stability *in vivo*, bioavailability and/or biological activity; such as:
- N- and C-terminus modifications to prevent exopeptidase degradation:
   o C-terminal amidation
   o N-terminal acetylation increases peptide lipophilicity,
- cyclization by forming a disulfide bridge,
- alkylation of amide nitrogen to prevent endopeptidase degradation,
- introduction of non-natural amino acids to modify the recognition site of the endopeptidase (2-methylalanine, alpha-dialkylated glycine, oligocarbamate, oligourea, guanidino or amidino backbones...),
- incorporation of non-genetically encoded amino acids (methylation, halogenation or chlorination of glycine or phenylalanine) into the CPP amino acid sequence,
- replacement of some or even all the L-amino acids with their corresponding D-amino acid or beta-amino acid analogues. Such peptides may be synthesized as "inverso" or "retro-inverso" forms, that is, by replacing L-amino acids of the sequence with D-amino acids, or by reversing the sequence of the amino acids and replacing the L-amino acids with D-amino acids. Structurally, the retro-inverse peptide is much more similar to the original peptide than the simple D-analogue. D-peptides are substantially more resistant to peptidases, and therefore are more stable in serum and tissues compared to their L-peptide counterparts. In a preferred embodiment CPPs containing L-amino acids are capped with a single D-amino acid to inhibit exopeptidase destruction,
- synthesis of CPP-derived oligocarbamate; the oligocarbamate backbone consists of a chiral ethylene backbone linked through relatively rigid carbamate groups (Cho et al., Science 261:1303-1305 (1993))

In another embodiment, the CPP contains contiguous or non- contiguous basic amino acid or amino acid analog, particularly guanidyl or amidinyl moieties. The terms "guanidyl" and "guanidine" are used interchangeably to refer to a moiety having the formula -HN=C(NH₂)NH (unprotonated form). As an example, arginine contains a guanidyl (guanidino) moiety, and is also referred to as 2-amino-5-guanidinovaleric acid or a-amino-6-guanidinovaleric acid. The terms "amidinyl" and "amidino" are used interchangeably and refer to a moiety having the formula -C(=NH)(NH2). A "basic amino acid or amino acid analog" has a side chain pKa of greater than 10. Preferred highly basic amino acids are arginine and/or lysine.

In a preferred embodiment, the CPP according to the invention comprises at least 1, 2, 3, 4, 5, 6, 7, 8, 9, 10 basic amino acid or amino acid analog, particularly lysine and arginine.

According to a more preferred embodiment, the CPP is further characterized by its ability to facilitate active transport across a biological membrane and/or to react with or bind to glycosaminoglycans (GAGs) (long unbranched molecules containing a repeating disaccharide unit) or specifically hyaluronic acid, heparin, heparan sulfate, dermatan sulfate, keratin sulfate or chondroitin sulfate and their derivatives. "Heparin, heparin sulfate or chondroitin sulfate derivatives" or "glycosaminoglycans" are understood to mean any product or sub-product as defined in the publications cited in references (Cardin and Weintraub, Arteriosclerosis 9: 21 (1989); Merton et al., Annu. Rev. Cell Biol. 8: 365 (1992); David, FASEB J. 7: 1023 (1993)).

The capacity of the CPPs to react with / bind to glycosaminoglycans (GAGs) can be determined by direct or indirect glycosaminoglycan-binding assays known in the art, such as the affinity coelectrophoresis (ACE) assay for peptide glycosaminoglycan binding described in the PCT patent application WO 00/45831. Several other methods well known in the art are available for analyzing GAG-peptides interactions, for example the method described in the PCT patent application WO 01/64738 or by , Weisgraber and Rall (J. Biol. Chem.262(33):11097-103) (specific example with the apolipoprotein B-100); or by a modified ELISA test: 96-well plates are coated with specific GAG (chondroitin sulfate A, B and C, heparin, heparin sulfate, hyaluronic acid, keratin sulfate, syndecan), peptide conjugated to a marker is then added for a defined time; after extensive washing, peptide binding is determined using specific analysis related to the marker.

CPPs capable of reacting *in vitro* and/or *in vivo* with glycosaminoglycans were described in the patent applications No WO 01/64738 and No WO 05/016960 and by De Coupade et al. (Biochem J. 390:407-18 (2005)). These peptides are amino acid sequences originating from human heparin binding proteins and/or anti-DNA antibodies selected from the group comprising: the lipoproteins such as human apolipoprotein B or E (Cardin et al., Biochem. Biosphys. Res. Com. 154: 741 (1988)), the agrine (Campanelli et al., Development 122: 1663-1672 (1996)), the insulin growth factor binding protein (Fowlkes et al., Endocrinol. 138: 2280-2285 (1997)), the human platelet-derived growth factor (Maher et al., Mol. Cell. Biol. 9: 2251-2253 (1989)), the human extracellular superoxide dismutase (EC-SOD) (Inoue et al., FEBS 269: 89-92 (1990)), the human heparin-binding epidermal growth factor-like growth factor (HB-EGF) (Arkonac et al., J. Biol. Chem. 273: 4400-4405 (1998)), the acid fibroblast growth factor (aFGF) (Fromm et al., Arch. Biochem. Bioph. 343: 92 (1997)), the basic fibroblast growth factor (bFGF) (Yayon et al. , Cell 64: 841-848 (1991)), the human intestinal mucin 2 sequence (Xu et al., Glyconjug J. 13: 81-90 (1996)), the human gamma interferon (Lortat-Jacob & Grimaud, FEBS 280: 152-154 (1991)), the subunit p40 of human interleukin 12 (Hasan et al., J. Immunol. 162: 1064-1070 (1999)), the factor 1-alpha derived from stromal cells (Amara et al., J. Biol. Chem. 272: 200-204 (1999)), the human neutrophil derived "heparin binding protein" (CAP 37/azurocidin) (Pohl et al., FEBS 272: 200-204 (1990)), an immunoglobulin molecule such as CDR2 and/or CDR3 regions of the anti-DNA monoclonal murine antibody F4.1 (Avrameas et al., Proc. Natl. Acad. Sci. 95: 5601 (1998)), the hyper variable CDR3 region of human anti-DNA monoclonal antibody RTT79 (Stevenson et al., J. Autoimmunity 6: 809 (1993)), the hyper variable area CDR2 and/or CDR3 of the human anti-DNA monoclonal antibody NE-1 (Hirabayashi et al., Scand. J. Immunol. 37: 533 (1993)), the hypervariable area CDR3 of the human anti-DNA monoclonal antibody RT72 (Kalsi et al., Lupus 4: 375 (1995)).

According to a more preferred embodiment, the CPP comprises an amino-acid sequence selected from the group consisting of a) (XBBBXXBX)n ; b) (XBBXBX)n ;c) (BBXmBBXp)n ; d) (XBBXXBX)n; e) (BXBB)n, f) (BmXX)n and g) (an antibody fragment), wherein B is a basic amino acid preferably lysine or arginine; X is a non-basic amino acid preferably hydrophobic amino acid, such as alanine, glutamic acid, isoleucine, leucine, methionine, phenylalanine, serine, tryptophan, tyrosine or valine; each m is independently an integer from zero to five; each n is independently an integer between one and ten; and each p is independently an integer between zero to five. In certain embodiments n may be 2 or 3 and X may be a hydrophobic amino acid. An antibody fragment is meant to include a less than full-length immunoglobulin polypeptide, e.g., a heavy chain, light chain, Fab, Fab2, Fv or Fc. The antibody can be for example human or murine. Preferably the antibody is an anti-DNA antibody. Preferably, the antibody fragment contains all or part of the CDR2 region of an antibody, particularly at least a portion of a CDR2 region of an anti-DNA antibody. Alternatively, the antibody contains all or part of the CDR3 region of an antibody, particularly at least a portion of a CDR3 region of an anti-DNA antibody. More specifically, the antibody fragment contains at least one CDR3 region of an anti-DNA human antibody, such as RTT79, NE-1 and RT72. Such antibody fragments have been described in PCT patent application n° WO 99/07414. More preferably the antibody has specific ligand-recognition (*i.e.* targeting) properties to achieve cell-type-specific nucleic acid delivery.

Preferably, the CPP according to the invention is further characterized in that it is originating from human proteins (*i.e.,* proteins naturally expressed by human cells). Thus, the characteristic of CPPs derived from human proteins compared to the CPPs derived from non-human proteins, is of primary interest in the planned use of these CPPs, since their immunogenicity is avoided or lowered. In addition, De Coupade et al., (Biochem J. 390:407-18 (2005)) have shown that human-derived peptides have low *in vivo* toxicity profiles consistent with their use as therapeutic delivery systems, unlike existing carrier peptides such as Tat peptides (Trehin and Merkle, Eur. J. Pharm. Biopharm. 58, 209-223 (2004)).

Among the CPPs described above, preferred are those capable of specifically penetrating into the cytoplasm, and in particular into the lysosomes of the cells. Penetration of a CPP into the lysosomes can be determined by various processes *in vitro* well known by one skilled in the art: for example by incubating the CPP with cells *in vitro*; then, the cells are incubated in the presence of specific anti-CPP labeled antibodies and specific anti-lysosomal protein labeled antibodies, followed by detection in the lysosome of the immunologic reaction between the CPP and the labeled antibodies. Another method is to conjugate the CPP to colloidal gold and incubate the conjugate with cells. The cells are then treated as usual for the electron microscope to visualise the intracellular localization.

Preferably, the CPP comprises an amino-acid sequence facilitating active transport across a biological membrane and has a length of more than 4 amino acids, preferably more than 6 amino acids. Preferably, it also has a length of less than 500 amino acids, preferably less than 25 amino acids.

Accordingly, preferred CPPs, derived from human heparin binding protein and capable of specifically penetrate into the cytoplasm of a target cell are selected from the group comprising:
- DPV3 (SEQ ID NO: 2): CPP reacting with heparin and dimer of a peptide derived from the C-terminal part of the sequence of human extracellular superoxide dismutase (EC-SOD) (Inoue et al., FEBS 269: 89-92 (1990)). Advantageously, the applicant showed that the covalently coupled DPV3 peptide is able to mediate mannose 6-phosphate receptor-independent delivery of an exogenous beta-glucuronidase in cell lysosomes *in vitro.* In addition, the applicant also showed that DPV3-enzyme conjugate significantly enhances the M6P/beta-glucuronidase intracellular delivery and allows an efficient decrease of GAG level in cells. DPV3 comprises an amino acid sequence of formula f) (BmXX)n wherein m = 6 and n = 2 and wherein X is selected from the group comprising glutamic acid and serine;;
- DPV6 (SEQ ID NO: 3): CPP reacting with heparin and derived from the amino acid sequence of the C-terminal part of chain A of the human platelet-derived growth factor (Maher et al., Mol. Cell. Biol. 9: 2251-2253 (1989)). DPV6 comprises an amino acid sequence of formula c) (BBXmBBXp)n wherein m = 0, p = 0 and n = 1;
- DPV7 (SEQ ID NO: 4) and DPV7b (SEQ ID NO: 5): CPPs reacting with heparin and derived from the C-terminal part of the sequence of the human heparin-binding epidermal growth factor-like growth factor (HB-EGF) (Arkonac et al., J. Biol. Chem. 273: 4400-4405 (1998)). DPV7 and DPV7b comprises an amino acid sequence of formula c) (BBXmBBXp)n wherein m = 0, p = 0 and n = 1;
- DPV3/10 (SEQ ID NO: 6) CPP reacting with heparin and derived from the C-terminal part of the sequence of human extracellular superoxide dismutase (EC-SOD) (see above) and from C-terminal part of the human intestinal mucin 2 sequence (Xu et al., Glyconjug J. 13: 81-90 (1996)). DPV3/10 comprises an amino acid sequence of formula c) (BBXmBBXp)n wherein m = 1, p = 2 and n = 1;
- DPV10/6 (SEQ ID NO 7) CPP reacting with heparin and derived from C-terminal part of the human intestinal mucin 2 sequence (see above) and from the C-terminal part of chain A of the platelet-derived growth factor (see above). DPV 10/6 comprises an amino acid sequence of formula c) (BBXmBBXp)n wherein m = 1, p = 2 and n = 1;

One particular interesting CPP is DPV3.

The CPP and the lysosomal enzyme can be conjugated / linked by chemical coupling in any suitable manner known in the art. Many known chemical cross-linking methods are non-specific, i. e., they do not direct the point of coupling to any particular site on CPP or the suitable lysosomal enzyme. As a result, use of non-specific cross-linking reagents may attack functional sites or sterically block active sites, rendering the conjugated proteins biologically inactive. The lysosomal enzyme can be coupled directly to the CPP either on one of those terminal ends (N or C terminus) or on a side chain or one of the amino acids. The lysosomal enzyme can also be coupled indirectly by a connecting arm either to one of the terminal ends of the peptides or to a side chain of one of the amino acids.

One way to increasing coupling specificity is to directly chemical coupling to a functional group found only once or a few times in one or both of the polypeptides to be cross-linked. For example, in many proteins, cysteine, which is the only protein amino acid containing a thiol group, occurs only a few times. Also, for example, if a polypeptide contains no lysine residues, a cross-linking reagent specific for primary amines will be selective for the amino terminus of that polypeptide. Successful utilization of this approach to increase coupling specificity requires that the polypeptide have the suitably rare and reactive residues in areas of the molecule that may be altered without loss of the molecule's biological activity.

Cysteine residues may be replaced when they occur in parts of a polypeptide sequence where their participation in a cross-linking reaction would otherwise likely interfere with biological activity. When a cysteine residue is replaced, it is typically desirable to minimize resulting changes in polypeptide folding. Changes in polypeptide folding are minimized when the replacement is chemically and sterically similar to cysteine. For these reasons, serine is preferred as a replacement for cysteine. As demonstrated in the examples below, a cysteine residue may be introduced into a polypeptide's amino acid sequence for cross-linking purposes. When a cysteine residue is introduced, introduction at or near the amino or carboxy terminus is preferred. Conventional methods are available for such amino acid sequence modifications, whether the polypeptide of interest is produced by chemical synthesis or expression of recombinant DNA.

Coupling of the two constituents can be accomplished via a cross-linking reagent. There are several intermolecular cross-linking reagents which can be utilized, see for example, Means and Feeney, CHEMICAL MODIFICATION OF PROTEINS, Holden-Day, 1974, pp. 39-43. Among these reagents are, for example, *N*-succinimidyl 3-(2-pyridyldithio) propionate (SPDP) or N, N'- (1,3-phenylene) bismaleimide (both of which are highly specific for sulfhydryl groups and form irreversible linkages); N, N'-ethylene-bis-(iodoacetamide) or other such reagent having 6 to 11 carbon methylene bridges (which relatively specific for sulfhydryl groups); and 1, 5-difluoro-2,4-dinitrobenzene (which forms irreversible linkages with amino and tyrosine groups). Other cross-linking reagents useful for this purpose include: p, p'-difluoro-N, N'-dinitrodiphenylsulfone (which forms irreversible cross-linkages with amino and phenolic groups); dimethyl adipimidate (which is specific for amino groups); phenol-1, 4-disulfonylchloride (which reacts principally with amino groups); hexamethylenediisocyanate or diisothiocyanate, or azophenyl-p-diisocyanate (which reacts principally with amino groups); glutaraldehyde (which reacts with several different side chains) and disdiazobenzidine (which reacts primarily with tyrosine and histidine).

Cross-linking reagents may be homobifunctional, *i. e*., having two functional groups (i.e. reactive groups) that undergo the same reaction. An example of homobifunctional cross-linking reagent is bismaleimidohexane ("BMH"). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of polypeptides that contain cysteine residues.

Cross-linking reagents may also be heterobifunctional. Heterobifunctional cross-linking reagents have two different functional groups, for example an amine-reactive group and a thiol-reactive group, that will cross-link two proteins having free amines and thiols, respectively. Preferred heterobifunctional cross-linking reagents are succinimidyl 4- (N-maleimidomethyl) cyclohexane-1-carboxylate ("SMCC"), N-maleimidobenzoyl-N-hydroxysuccinimide ester ("MBS"), and succinimide 4- (p-maleimidophenyl) butyrate ("SMPB"), an extended chain analog of MBS. The succinimidyl group of these cross-linking reagents reacts with a primary amine forming an amide bond, and the thiol-reactive maleimide forms a covalent thioether bond with the thiol of a cysteine residue.

Cross-linking reagents often have low solubility in water. A hydrophilic moiety, such as a sulfonate group, may be added to the cross-linking reagent to improve its water solubility. Sulfo-MBS and sulfo-SMCC are examples of cross-linking reagents modified for water solubility.

Many cross-linking reagents yield a conjugate that is essentially non-cleavable under cellular conditions. However, some cross-linking reagents contain a covalent bond, such as a disulfide, that is cleavable under cellular conditions. For example, Traut's reagent, dithiobis(succinimidylpropionate) ("DSP"), and N-succinimidyl 3- (2-pyridyldithio) propionate ("SPDP") are well-known cleavable cross-linking reagents. Another example is the hydrazine derivatives such as the 4-(-4-*N*-Maleimidophenyl)butyric acid hyrazide (MPBH), 4-(*N*-Maleimidomethyl)cyclohexane-1-carboxyl-hydrazide (M₂C₂H), or the 3-(2-Pyridyldithio)propionyl hydrazide (PDPH). The use of a cleavable cross-linking reagent permits the lysosomal enzyme to separate from the CPP after delivery into the target cell. Direct disulfide linkage may also be useful.

Numerous cross-linking reagents, including the ones discussed above, are commercially available. Detailed instructions for their use are readily available from the commercial suppliers. A general reference on protein cross-linking and conjugate preparation is: Wong, CHEMISTRY OF PROTEIN CONJUGATION AND CROSS-LINKING, CRC Press (1991).

Chemical cross-linking may include the use of spacer arms. Spacer arms provide intramolecular flexibility or adjust intramolecular distances between conjugated domains and thereby may help preserve biological activity. A spacer arm may be in the form of a polypeptide domain that includes spacer amino acids, e. g. proline. Alternatively, a spacer arm may be part of the cross-linking reagent, such as in "long-chain SPDP" (Pierce Chem. Co.,Rockford, IL. , cat. No. 21651 H).

The chimeric polypeptide may be linked to one or more additional domains. For example, the chimeric polypeptide may additionally be linked to a GST (glutathione S-transferase) protein in which the chimeric polypeptide is fused to the C-terminus of the GST sequences. Such fusion proteins can facilitate the purification of chimeric polypeptide.

In one embodiment of the invention, the CPP is coupled to the lysosomal enzyme by at least one molecule (called an "anchoring molecule") that has a strong natural affinity for the lysosomal enzyme. The natural affinity of the anchoring molecule for the lysosomal enzyme allows the CPP to interact non-covalently with the lysosomal enzyme, and hence to carry it along in intracellular travel. Another especially interesting advantage of this type of coupling consists of the fact that, due to the natural affinity of the anchoring molecule for the lysosomal enzyme, these two elements are coupled in a totally natural way, with no chemical or biochemical interaction.

Alternatively, the chimeric polypeptide can be produced by genetic engineering as a fusion polypeptide that includes the CPP and the suitable lysosomal enzyme sequence which can conveniently be expressed in known suitable host cells. Fusion polypeptides, as described herein, can be formed and used in ways analogous to or readily adaptable from standard recombinant DNA techniques. Accordingly, the present invention provides nucleic acid molecules and expression vectors comprising a nucleic acid sequence encoding a lysosomal enzyme and a CPP of the invention, see below. There are an abundance of expression vectors available and one skilled in the art could easily select an appropriate vector. In addition, standard laboratory manuals on genetic engineering provide recombinant DNA methods and methods for making and using expression vectors. For example, use of recombinant gene delivery vectors for treating or preventing LSDs are described in PCT patent applications WO 00/73482 and WO 02/055684.

If desired, one or more amino acids can additionally be inserted between the first peptide domain comprising the CPP and the second polypeptide domain comprising the suitable lysosomal enzyme. In some embodiments, the first or second domain includes a sequence that facilitates association of the CPP with the lysosomal enzyme.

The scope of the invention extends to the use of a chimeric polypeptide of the invention for the manufacture of a medicament for treating or preventing lysosomal storage disorders (LSDs).

The invention also provides a method of treating or preventing lysosomal storage disorders (LSDs) by administering to a subject in which such treatment or prevention is desired a composition comprising a chimeric polypeptide of the invention in an amount sufficient to treat or prevent the disease in the subject. Any composition can comprise an effective amount of a chimeric polypeptide of the invention from 0.1 % to 99%, preferably 1% to 70%, of the composition. As example, the dosages of the chimeric polypeptide of the invention, when they are used for the effects indicated, will be between around 0.05 and 1,000 mg, and preferably 0.5, 1.0, 2.5, 5.0, 10.0, 15.0, 25.0, 50.0, 100.0, 250.0, 500.0 mg per composition.

Efficaciousness of treatment is determined in association with any known method for diagnosing or treating LSDs. Based on the selected enzymatic experimental model to describe the invention, the invention provides a method of treating or preventing mucopolysaccharidosis type VII (or MPS VII) by administering to a subject in which such treatment or prevention is desired a composition comprising a CPP capable of reacting with or binding to glycosaminoglycans (GAGs) fused or conjugated to beta-glucuronidase or a functional analog thereof in an amount sufficient to treat or prevent the disease in the subject. The subject can be any mammal, e. g., a human, a primate, mouse, rat, dog, cat, cow, horse, Pig.

The chimeric polypeptides, or nucleic acid molecules encoding these chimeric polypeptides (also referred to herein as "therapeutics" or "active compounds") of the invention, and derivatives, fragments, analogs and homologs thereof, can be incorporated into pharmaceutical compositions suitable for administration ; such compositions comprising a pharmaceutically acceptable carrier.

As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and nonaqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e*. *g*., intravenous, intradermal, subcutaneous, oral (*e. g*., inhalation), transdermal (*i. e*., topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic. Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, CremophorEL (BASF, Parsippany, N. J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (e. g., glycerol, propylene glycol, and liquid polyethylene glycol, and the like), and suitable mixtures thereof.

The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. For administration by inhalation, the compounds are delivered in the form of an aerosol spray from pressured container or dispenser which contains a suitable propellant, e. g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compositions can also be prepared in the form of suppositories (*e. g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

In some embodiments, oral or parenteral compositions are formulated in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (see, *e. g*., U. S. Patent No. 5,328, 470) or by stereotactic injection (see, *e. g*., Chen, et al., 1994. Proc. Natl. Acad. Sci. USA 91 :3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e. g*., retroviral vectors, the pharmaceutical preparation can include one or more cells that produce the gene delivery system.

Sustained-release preparations can be prepared, if desired. Suitable examples of sustained-release preparations include semi-permeable matrices of solid hydrophobic polymers containing the active compound, which matrices are in the form of shaped articles, *e. g*., films, or microcapsules. Examples of sustained-release matrices include polyesters, hydrogels (for example, poly (2-hydroxyethyl-methacrylate), or poly (vinylalcohol)), polylactides (U. S. Pat. No. 3,773, 919), copolymers of L-glutamic acid and y ethyl-L-glutamate, non-degradable ethylene-vinyl acetate, degradable lactic acid-glycolic acid copolymers such as the LUPRON DEPOT (injectable microspheres composed of lactic acid-glycolic acid copolymer and leuprolide acetate), and poly-D- (-)-3-hydroxybutyric acid.

As examples, the oral dosages of the chimeric polypeptides of the invention, when they are used for the effects indicated, will be between around 0.05 and 1,000 mg/day by the oral route and, preferably come in the form of tablets containing 0.5, 1.0, 2.5, 5.0, 10.0, 15.0, 25.0, 50.0, 100.0, 250.0, 500.0 and 1,000.0 mg of active ingredient. The effective plasma levels of the vectors or transporters loaded with at least one substance of interest will range from 0.002 mg to 50 mg per kilogram of body weight and per day.

The chimeric polypeptides or nucleic acid encoding the chimeric polypeptides of the invention may be administered in the form of single daily doses, or the total daily dose may be administered in two, three or four doses per day.

The pharmaceutical compositions can be included in a container, kit, pack, or dispenser together with instructions for administration.

The invention is therefore aimed at supplying CPPs such as the those described above, characterized by the fact that it can incorporate a substance of interest such as a lysosomal enzyme (for example beta-glucuronidase) into the lysosomes. More specifically, the subject of the invention is a CPP whose penetration into lysosomes is quite independent from the nature of the substance of interest that is coupled to it.

Another aspect of the invention pertains to vectors, preferably expression vectors, containing a nucleic acid encoding a fused polypeptide of the invention, or derivatives, fragments, analogs or homologs thereof. As used herein, the term "vector" refers to a nucleic acid molecule capable of transporting another nucleic acid to which it has been linked. One type of vector is a "plasmid", which refers to a linear or circular double stranded DNA loop into which additional DNA segments can be ligated. Another type of vector is a viral vector, wherein additional DNA or RNA segments can be ligated into the viral genome. Certain vectors are capable of autonomous replication in a host cell into which they are introduced (e. g., bacterial vectors having a bacterial origin of replication and episomal mammalian vectors; yeast vectors). Other vectors (*e. g*., non-episomal mammalian vectors) are integrated into the genome of a host cell upon introduction into the host cell, and thereby are replicated along with the host genome.

Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e. g., replication defective retroviruses, adenoviruses and adeno-associated viruses), which serve equivalent functions. Additionally, some viral vectors are capable of targeting a particular cells type either specifically or non-specifically.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively linked to the nucleic acid sequence to be expressed. Within a recombinant expression vector, "operably linked" is intended to mean that the nucleotide sequence of interest is linked to the regulatory sequence (s) in a manner that allows for expression of the nucleotide sequence (*e. g*., in an *in vitro* transcription/translation system or in a host cell when the vector is introduced into the host cell). The term "regulatory sequence" is intended to includes promoters, enhancers and other expression control elements (*e. g*., polyadenylation signals). Such regulatory sequences are described, for example, in Goeddel; GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e. g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e. g*., chimeric polypeptides, mutant forms of the chimeric polypeptide, fusion proteins, etc.).

The recombinant expression vectors of the invention can be designed for expression of the chimeric polypeptide in prokaryotic or eukaryotic cells. For example, the chimeric polypeptide can be expressed in bacterial cells such as *E*. *coli,* insect cells (using baculovirus expression vectors) yeast cells, plant cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase. Expression of proteins in prokaryotes is most often carried out in *E*. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (1) to increase expression of recombinant protein; (2) to increase the solubility of the recombinant protein; and (3) to aid in the purification of the recombinant protein by acting as a ligand in affinity purification. Often, in fusion expression vectors, a proteolytic cleavage site is introduced at the junction of the fusion domain and the recombinant protein to enable separation of the recombinant protein from the fusion domain subsequent to purification of the fusion protein. Such enzymes, and their cognate recognition sequences, include Factor Xa, thrombin and enterokinase. Typical fusion expression vectors include pGEX (Pharmacia Biotech Inc ; Smith and Johnson, Gene 67: 31-40 (1988)), pMAL (New England Biolabs, Beverly, Mass.) and pRIT5 (Pharmacia, Piscataway, N. J.) that fuse glutathione S-transferase (GST), maltose E binding protein, or protein A, respectively, to the target recombinant protein.

Examples of suitable inducible non-fusion *E*. *coli* expression vectors include pTrc (Amrann et al., Gene 69: 301-315 (1988)) and pET 11d (Studier et al., GENE EXPRESSION TECHNOLOGY: METHODS IN ENZYMOLOGY 185, Academic Press, San Diego, Calif. 60-89 (1990)).

One strategy to maximize recombinant protein expression in *E*. *coli* is to express the protein in a host bacteria with an impaired capacity to proteolytically cleave the recombinant protein. See, Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in E. *coli* (Wada et. al., Nucleic Acids Res. 20: 2111-2118 (1992)). Such alteration of nucleic acid sequences of the invention can be carried out by standard DNA synthesis techniques.

In another embodiment, the chimeric polypeptide expression vector is a yeast expression vector. Examples of vectors for expression in yeast *S. cerevisiae* are well known in the art.

Alternatively, the chimeric polypeptide can be expressed in insect cells using baculovirus expression vectors.

In yet another embodiment, a nucleic acid of the invention is expressed in mammalian cells using a mammalian expression vector. When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells. See, *e*. *g.,* Chapters 16 and 17 of Sambrook et al., MOLECULAR CLONING: A LABORATORY MANUAL. 3rd., Cold Spring Harbor Laboratory, Cold Spring Harbor Laboratory Press, Cold Spring Harbor, N. Y., (2001).

In another embodiment, the recombinant mammalian expression vector is capable of directing expression of the nucleic acid preferentially in a particular cell type (*e. g.*, tissue-specific regulatory elements are used to express the nucleic acid). Tissue-specific regulatory elements are known in the art.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein. Additionally, host cells could be modulated once expressing the chimeric polypeptide, and may either maintain or loose original characteristics.

A host cell can be any prokaryotic or eukaryotic cell. For example, chimeric polypeptide can be expressed in bacterial cells such as *E*. *coli,* insect cells, yeast or mammalian cells (such as Chinese hamster ovary cells (CHO) or COS cells). Alternatively, a host cell can be a premature mammalian cell, *i. e.,* pluripotent stem cell. A host cell can also be derived from other human tissue. Other suitable host cells are known to those skilled in the art.

Vector DNA can be introduced into prokaryotic or eukaryotic cells via conventional transformation, transduction, infection or transfection techniques. As used herein, the terms"transformation""transduction","infection"and"transfection"are intended to refer to a variety of art-recognized techniques for introducing foreign nucleic acid (*e. g*., DNA) into a host cell, including calcium phosphate or calcium chloride co-precipitation, DEAE-dextran-mediated transfection, lipofection, or electroporation. In addition transfection can be mediated by a transfection agent. By "transfection agent" is meant to include any compound that mediates incorporation of DNA in the host cell, *e. g*., liposome. Suitable methods for transforming or transfecting host cells can be found in Sambrook, et al. (MOLECULAR CLONING: A LABORATORY MANUAL. 3rd, Cold Spring Harbor Laboratory, Cold Spring Harbor Laboratory Press, Cold Spring Harbor, N. Y. , (2001)), and other laboratory manuals.

Transfection may be "stable" (*i. e*. intergration of the foreign DNA into the host genome) or "transient" (*i. e*., DNA is episomally expressed in the host cells).

For stable transfection of mammalian cells, it is known that, depending upon the expression vector and transfection technique used, only a small fraction of cells may integrate the foreign DNA into their genome the remainder of the DNA remains episomal. In order to identify and select these integrants, a gene that encodes a selectable marker (*e. g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding or can be introduced on a separate vector. Cells stably transfected with the introduced nucleic acid can be identified by drug selection (*e. g*., cells that have incorporated the selectable marker gene will survive, while the other cells die).

In another embodiment the cells modulated by the chimeric polypeptide or the transfected cells are identified by the induction of expression of an endogeneous reporter gene. In a specific embodiment, the promoter is the lysosomal enzyme promoter driving the expression of green fluorescent protein (GFP).

### BRIEF DESCRIPTION OF THE DRAWINGS

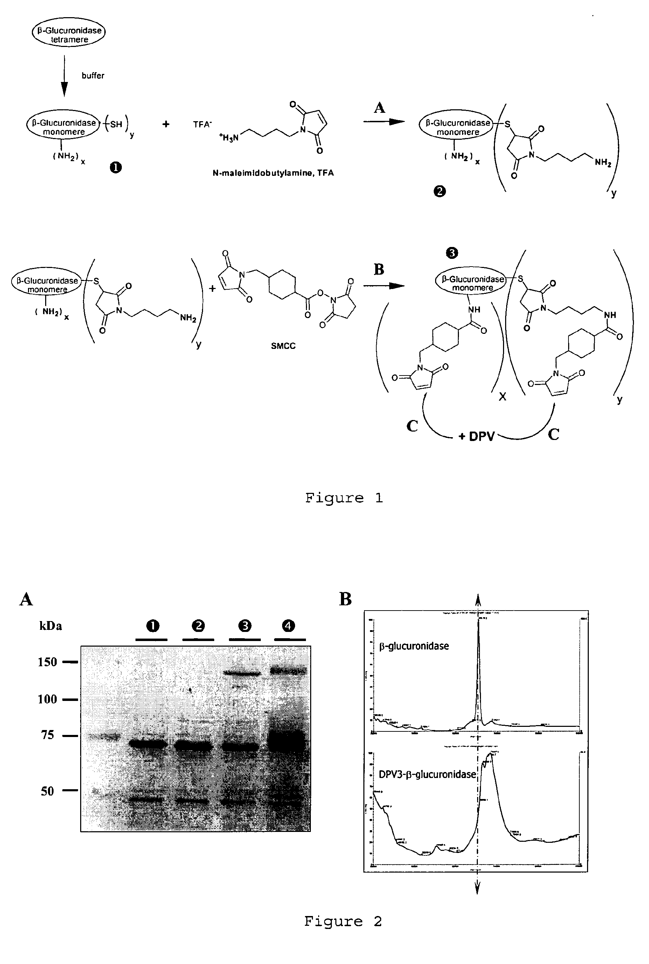

Figure 1 schematically shows the chemical conjugation of a CPP (*e. g*. DPV) on beta-glucuronidase enzyme.
Figure 2 shows the analysis of DPV3-beta-glucuronidase conjugate. A: SDS-Page analysis of beta-glucuronidase (①), beta-glucuronidase-Mal-NH₂ intermediate (②), beta-glucuronidase-Mal-NH-SMCC intermediate (③) and beta-glucuronidase-Mal-NH-SMCC-DPV3 (DPV3-beta-glucuronidase) (④). Proteins were separated on 8% gel under reducing conditions. The gel was stained with Coomassie blue as described in the Materials and Methods. **B:** MALDI-TOF mass spectra analysis of beta-glucuronidase and DPV3-beta-glucuronidase. The mass range from 38 to 98 kDa is expected.

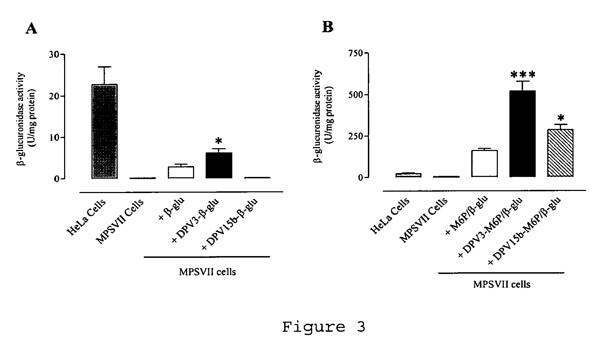

Figure 3 shows beta-glucuronidase activities in MPSVII cytoplasm extracts. MPSVII cells were untreated (control) or incubated with (A) non-phosphomannosylated beta-glucuronidase (beta-Glu) or DPV(3 or 15)-beta-glucuronidase conjugates (50µg/mL); or (B) phosphomannosylated beta-glucuronidase (M6P/beta-Glu) or DPV(3 or 15)-beta-glucuronidase conjugates (20µg/mL) as indicated for 16-18 hours at 37°C. HeLa cells were used as internal control. Values are expressed as the number of nmoles of hydrolyzed substrate (MUGluc) per hour per mg of extract protein and represent the mean of more than three experiments ± SEM. *: Significant differences as compared with unconjugated enzyme (p<0.05). ***: Significant differences as compared with unconjugated enzyme (p<0.001).

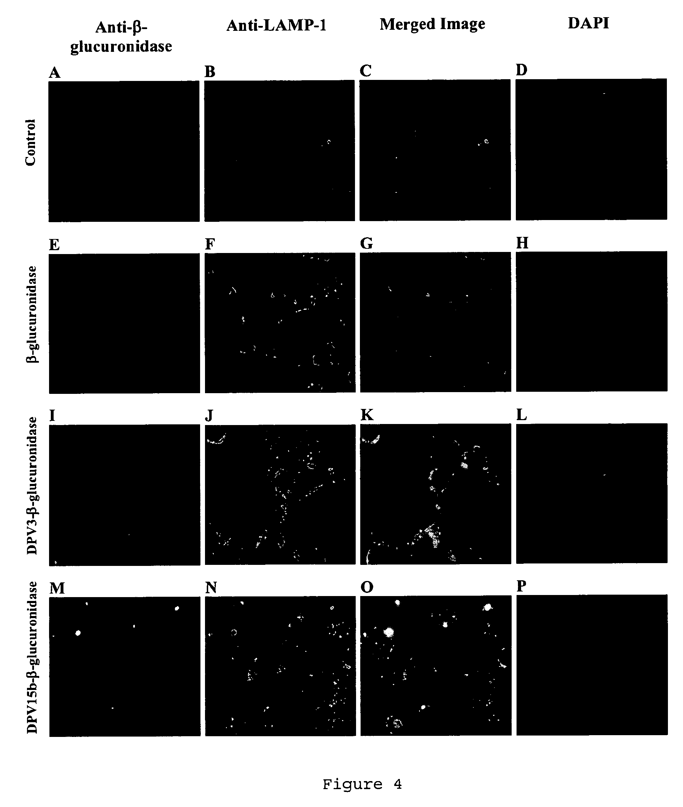

Figure 4 shows the uptake and subcellular localization of DPV3- and DPV15b-beta-glucuronidase. MPSVII cells were untreated (control) or incubated with protein (50µg/mL) as indicated to the left of the panel for 4 hours at 37°C. Cells were then fixed and stained with anti-beta-glucuronidase pAb (*A, E, I,* and *M*) and anti-LAMP-1 mAb (*B, F, J,* and *N*) followed by incubation with TRITC-labeled anti-rabbit IgG antibody (*A, E, I,* and *M*) and FITC-labeled anti-rat IgG antibody (*B, F, J,* and *N*). Merged images are shown in the third column and DAPI counter-staining is shown in the fourth column.

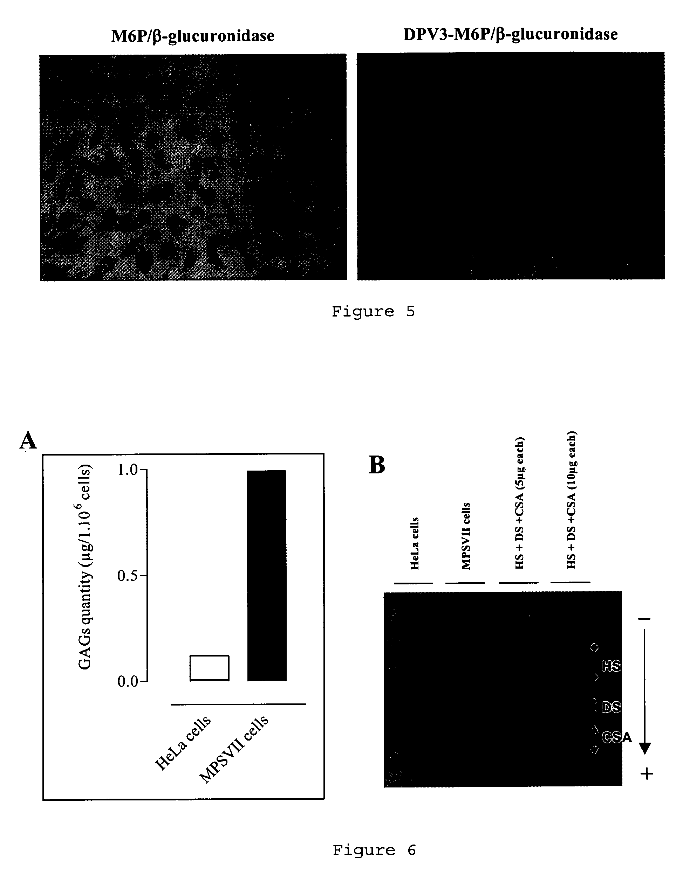

Figure 5 shows the intracellular localization and activity of M6P/beta-glucuronidase and DPV3-M6P/beta-glucuronidase in MPSVII cells. Cells were incubated with both enzymes for 4 h at 37°C; they were washed, fixed with 0.1% glutaraldehyde and incubated with X-gluc. Beta-glucuronidase activity was visualized by the deposition at the site of enzymatic cleavage, of the water-insoluble indoxyl molecules dimmer (indigo dye). Untreated cells were negative (data not shown). Microscopy amplification: X40.
Figure 6 shows sulfated GAGs content of MPSVII cells. Sulfated GAGs from HeLa and MPSVII cells were extracted as described in Materials and Methods. A: Sulfated GAGs were quantified in extract from about 2.10⁶ cells by using dimethylene blue assay as described in materials and Methods. **B:** Sulfated GAGs extract from about 7.10⁶ cells as well as a mixture of standard GAGs containing 5 or 10µg each of chondroitin 4-sulfate (CSA), dermatan sulfate (DS) and heparan sulfate (HS) were applied to a 0.5% agarose gel and run for 2 h at 50 V at 4°C in 1,3-diaminopropane/acetate buffer (pH 9.0). Sulfated GAGs in the gel were fixed with 0.1% *N*-cetyl-*N,N,N*-trimetylammonium bromide in water. After 12 h, the gel was dried and stained with 0.1 % toluidine blue in acetic acid/ethanol/water (0.1:5:5, V/V).

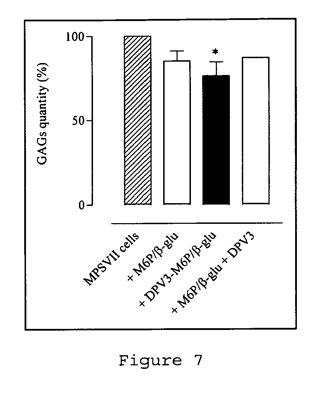

Figure 7 shows the quantity of GAG (%) in MPSVII cells following different treatments. GAG content of MPSVVII cells is considered as 100%. MPSVII cells were incubated with or without enzyme (5µg/mL) for 6 h at 37°C. Intracellular GAGs were extracted and quantified with a dimethylene blue dye-binding assay. Results were expressed relative to untreated MPSVII cells defined as 100% for the quantity of GAGs. Statistical Dunnett's analysis as compared with MPSVII cells GAGs content: *p<0.05.

### EXAMPLES

Other advantages and characteristics of the invention will appear from the following examples which refer to the above figures. The examples are given to illustrate the invention but not to limit the scope of the claims.

### I- Materials and Methods

### I-1 Compounds

### 1-1-1 Peptides

DPV3 peptide (SEQ ID N°2) (manufactured by BACHEM). This CPP is known to transport reporter proteins to the cytoplam (De Coupade et al., Biochem J. 390:407-18 (2005)).

DPV15b peptide (SEQ ID N°12) (manufactured by Neosystem). This CPP is known to transport reporter proteins to the nucleus (De Coupade et al., Biochem J. 390:407-18 (2005)).

### I-1-2 Enzymes

Non-phosphorylated enzyme: beta-glucuronidase (beta-glu) from *Escherichia coli*, Type VII-A (Sigma #G7646). This enzyme shows 47% of homology with human protein.

Phosphorylated enzyme: M6P-beta-glucuronidase (M6P/beta-glu) from bovine liver (GLYKO #GKGAG-5007).

### I-1-3 Cell Lines

HeLa cells (ATCC #CCL-2): *Human epithelial cells from uterine adenocarcinoma.* Cells were cultured in DMEM (Gibco BRL) + 2mM L-glutamine + 1 mM Sodium pyruvate + 10% heat-inactivated fetal calf serum (FCS).
NIH/3T3 cells (ATCC #CRL-1658): *Immortalized fibroblast from a NIH Swiss mouse embryo.* Cells were cultured in DMEM (Gibco BRL) + 2mM L-glutamine + 1 mM Sodium pyruvate + 10% heat-inactivated fetal calf serum (FCS).
MPSVII cells: *AgT (SV40)-transformed fibroblast from murine model of MPS-VII.* These cells are beta-glucuronidase deficient fibroblasts and were provided by Dr J.M. Heard (Institut Pasteur, Paris, France). Cultures were maintained in DMEM (Gibco BRL) + 2mM L-glutamine + 1 mM Sodium pyruvate + 10% heat-inactivated fetal calf serum (FCS) + 1mg/mL G418 (Sigma).

### 1-2 Chemical Conjugation of DPV onto enzyme

The same three-step conjugation strategy was used for both proteins and both peptides (Figure 1). The first step allowed masking of accessible thiol functions with the cross-linking reagent N-maleimidobutylamine. The heterobifunctional cross-linking reagent Succinimidyl-4-(N-maleimidomethyl)cyclohexane-1-carboxylate (SMCC) was then allowed to react with the available lysine amines on the enzyme and the linker amine of the N-maleimidobutylamine. After elimination of excess cross-linking reagent by dialfiltration, the DPV peptide was then added, its thiol function (N- or C-terminal cystein) reacting with the maleimide moiety of SMCC. The cross-linked conjugates were analysis by SDS/PAGE (8%) - Coomassie blue R-250 staining, MALDI-TOF and assay for beta-glucuronidase activity (see paragraph 1-3 below) (see Figure 2).

### I-3 In Vitro Lysosomal Enzyme Uptake Assays

Beta-glucuronidase deficient cells (MPSVII cells) were plated onto 6-well culture dishes and allowed to settle for 24 hours (80% confluence). Prior to the addition of the enzyme (i.e. beta-glucuronidase conjugated to a DPV or not), the MPSVII cells were washed once with preheated DMEM. The enzyme was added to the culture media containing 10% FCS at a concentration of 20 µg/mL for phosphorylated enzyme (1400 units/mL) and 50 µg/mL for no phosphorylated enzyme (1500 units/mL). After a 16-18-h incubation at 37°C, the media was then removed and the cells washed twice with DMEM. The cells were then harvested and lysed in 30 µL of lysis buffer (10 mM Tris-HCl, pH 7.4; 1 mM EDTA, pH 8.0; 150 mM NaCl; 1% Triton X100; 0.1% SDS; 1 mM PMSF; 2 µg/mL aprotinin; 10 µg/mL leupeptin; 1 µg/mL pepstatin) for 30 min at 4°C. The cellular debris were removed by centrifugation at 12000g and 4°C for 15 min and the protein concentration in the supernatants was determined using the Bio-Rad Protein Assay, based on the method of Bradford with bovine serum albumin as a standard. Beta-glucuronidase activity in the supernatants was quantified using "FluorAce^{™} beta-glucuronidase Reporter Assay Kit" (BIO-RAD #170-3151), based on the hydrolysis of 4-methylumbelliferyl-*beta*-D-glucuronide (MUGluc) substrate. Fluorescence was measured using fluorometer (355 nm excitation, 460 nm emission). Fluorescence values were used to calculate units with a calibration curve of 4-methylumbelliferone fluorescence. One unit of beta-glucuronidase activity was defined as 1 nanomole of 4-methylumbelliferone released per hour. The specific beta-glucuronidase activity was expressed in beta-glucuronidase unit per mg of protein.

### 1-4 Qualitative and Quantitative Analysis of Glycosaminoglycans (GAGs)

Cells were plated onto 6-well culture dishes and allowed to settle for 24 hours (80% confluence) in their culture media. Prior to the addition of the enzyme (*i.e.* beta-glucuronidase conjugated to a DPV or not), the cells were washed once with pre-heated DMEM. Protein was added to the culture media containing 1% FCS at a concentration of 5 µg/mL for phosphorylated enzyme and 50 or 100 µg/mL for no phosphorylated enzyme. After a 6 hour incubation at 37°C, the media was then removed and the cells washed twice with DMEM. The cells were then harvested, washed with PBS and counted in Kova-slide. 6.75x10⁶ cells for qualitative analysis and 5x10⁶ cells for quantitative analysis were recovered and centrifuged at 1200 rpm for 5 min at room temperature. Cell pellets were incubated overnight with papain (2mg/mL in 0.1 M Sodium phosphate buffer, pH 6.5, containing 5 mM L-Cystein-HCl and 10 mM EDTA) at 60°C. The reaction was stopped by heating at 100°C for 5 min. The samples were cooled to room temperature. Afterwards, debris were removed by centrifugation at 13000 rpm for 30 min. in a micro-centrifuge (Biofuge-pico, Heraeus Instruments Inc., Newtown, CT). Trichloroacetic acid and NaCl were added to the supernatant up to 10% and 1 M final concentration, respectively. The precipitate formed was removed by centrifugation at 13000 rpm for 30 min. in micro-centrifuge Biofuge-pico. The GAGs were precipitated from the supernatant by the slow addition of 2 volumes of ethanol whilst shaking. After 24 h at -20°C, the precipitate was collected by centrifugation (19000g) at 4°C for 1 h, vacuum dried, resuspended in 20µL of a solution containing deoxyribonuclease I (0.05 mg/mL), 10 mM Tris-HCl pH 7.4, 25mM MgCl₂ and 5 mM CaCl₂, and incubated at 37°C for 4 h.

GAGs were qualitatively analyzed using agarose gel electrophoresis, as described (Pavão et al., 1998; Oba-Shinjo et al., 2003). Sample was mixed with 0.1 volume of loading solution (electrophoresis buffer/50% glycerol) and immediately layered under the electrophoresis buffer in the preformed well. GAGs were applied to an agarose gel (0.5%, W/V) and run in 0.05 M 1,3-diaminopropane/acetate buffer (pH 9.0) at 50 V for 2 h at 4°C. GAGs in the gel were fixed with 0.1 % *N*-cetyl-*N,N,N-*trimetylammonium bromide in water for 12 h. The gel was dried and stained with 0.1% toluidine blue in acetic acid/ethanol/water (0.1:5:5, V/V) for 24 h minimum. The gel was washed in acetic acid/ethanol/water (0.1:5:5, V/V) for 15 min and dried. Standard GAG markers from Sigma were used (heparan sulfate from bovine kidney [#H7640], dermatan sulfate or chondroitin sulfate B from porcine intestinal mucosa [#C3788] and chondroitin sulfate A or chondroitin 4-sulfate [#C8529]).

Sulphated GAGs concentration in the papain/DNAse I digests was measured by a modification of the 1,9-dimethylmethylene blue (DMMB) dye binding assay of Farndale et al. (1986). The color reagent was prepared by dissolving 8 mg 1,9-dimethylmethylene blue in 500 mL water containing 1.52 g glycine, 1.185 g NaCl and 47.5 mL 0.1 M HCl to give a solution at pH 3.0, with A₅₂₅ 0.31. The reagent was stored at room temperature in a brown bottle. Samples were diluted three-fold in H₂O. Duplicate 30 µL of each sample was mixed with 1.25 mL DMMB color reagent and was read immediately at an absorbance of 525 nm. The samples were measured against blank sample and known standards of shark chondroitin-4 sulfate (0.5 to 10 µg/mL) in the same solvent as the samples. Results were expressed relative to untreated MPSVII cells defined as 100% for the quantity of GAGs.

### 1-5 Chromogenic Staining of Cells in Culture

A histochemical assay for beta-glucuronidase was performed according to the method described previously (Brusselbach, 2004). MPSVII cells were grown on permanox Lab-Tek 8-chamber slides and allowed to settle for 24 hours. Prior to the addition of the enzyme (DPV-beta-glucuronidase conjugate), the cells were washed once with preheated DMEM and incubated with phosphorylated protein (10µg/mL) for 4 hours at 37°C in DMEM containing 2mM L-glutamine, 1 mM Sodium pyruvate, 10% FCS and 1mg/mL G418. Then, cells were washed twice with PBS, fixed by incubation with 0.1% glutaraldehyde in PBS for 10 min at room temperature. Cells were rinsed three time with PBS, covered with a minimal volume of staining solution (0.1M sodium acetate buffer, pH 5; 3mM potassium ferricyanide; 3mM potassium ferrocyanide; 0.08% 5-bromo-4-chloro-3-indolyl-beta-D-glucuronide cyclohexylammonium (X-Gluc)) and incubated at 37°C for 2-24 h. The histochemical beta-glucuronidase reaction with X-Gluc produces the indigogenic blue precipitation. In order to terminate the reaction, the staining solution was removed and cells were rinsed twice with PBS. Cells were fixed for 15 min with 3.7% formaldehyde/PBS. After extensively washing with PBS, a drop of PBS/50% Glycerol solution was placed on a slide and sealed under a cover slip. The chromogenic stained cells were examined using an optical Leica microscope under normal light (20X or 40X lens). Picture was taken with a Nikon coolpix numeric camera, maximum zoom and a 0.63 X adaptator.

### I-6 Indirect Immunofluorescence Staining

MPSVII cells were grown on permanox Lab-Tek 8 chambers slides and incubated with non-phosphorylated protein for 4 hours at 37°C. Then, cells were washed twice with cold PBS, fixed and permeabilized by incubation in methanol/acetone solution (3V/7V) for 10 min at -20°C. Slides were air-dried. After blocking with PBS containing 2 mg/mL BSA (Sigma) for 30 min, the cells were incubated at room temperature for 1 hour with rabbit anti-bacteria beta-glucuronidase antiserum (Molecular Probes - #A-5790) and with rat anti-mouse LAMP-1 monoclonal antibody (BD Biosciences - #G-3060-05). Both primary antibodies were diluted 1:200 in PBS containing 2 mg/mL BSA. Cells were washed extensively in PBS containing 2 mg/mL BSA and incubated for 30 min at room temperature with secondary antibodies at a concentration of 7.5 µg/mL in PBS containing 2 mg/mL BSA: TRITC-conjugated donkey anti-rabbit IgG (Jackson ImmunoResearch - #711-025-152) and FITC-conjugated donkey anti-rat IgG (Jackson ImmunoResearch - #712-095-150). After extensively washing with PBS, a drop of Slow Fade Light Antifade kit solution with DAPI (Molecular Probes) was placed on a slide and sealed under a cover slip. The cell nuclei were visualized by DAPI counter-staining. The immunostained cells were examined using a fluorescent optical Leica microscope (40X or 63X lens) with the appropriate filter combination. Pictures were taken with a Nikon coolpix digital camera, maximum zoom and a 0.63 X adaptator. For double staining experiments, identical optical sections are presented.

### 1-7 Statistical Analysis

The statistical analyses (Dunnett test) were assessed using the GraphPad® prism 3.02 software.

### II Results

### II-1 Chemical conjugation

DPV3 or DPV15b peptide was conjugated on beta-glucuronidase (beta-glu) or M6P/beta-glucuronidase (M6P/beta-glu) as described in Material and Methods. Each conjugate was characterized by SDS-PAGE analysis (figure 2A). Up to 80% of DPV-enzyme conjugate was obtained whatever DPV or enzyme sample. Maldi Tof analyses were performed to check the number of DPV per enzyme monomer and showed that on average, one DPV sequence was conjugated per enzyme monomer (figure 2B). The enzyme activity was checked to verify that it was not affected by the conjugation. All conjugated enzymes were shown to retain full activity (data not shown). Beta-glu enzyme and DPV-beta-glu enzyme conjugate showed an activity of 30,000 units per mg of protein. M6P/beta-glu and DPV-M6P/beta-glu enzyme showed an activity of 70,000 units per mg of protein.

### II-2 DPV Mediates beta-glucuronidase Uptake by MPSVII Cells

### II-2-1 Quantitative Analysis of Enzyme Uptake

In order to evaluate the capacity of the DPV to allow and/or enhance enzyme internalization, the uptake of DPV-beta-glucuronidase and DPV-M6P/beta-glucuronidase by MPSVII cells was investigated. To this end, protein was exogenously added to the culture medium (~1500 units/mL), and uptake was analyzed by quantifying beta-glucuronidase activity in cytoplasmic cell extracts.

Both beta-glucuronidase and the DPV3-beta-glucuronidase conjugate were found to be internalized but the incorporated enzyme activity was three to four times higher in the DPV3-beta-glucuronidase conjugate compared to that of the unconjugated enzyme (Figure 3A). However, the activity level detected in HeLa cells was never reached. Following DPV3-beta-glucuronidase conjugate treatment of MPSVII cells, beta-glucuronidase activity was found at about 30% of the normal level in HeLa cells. For the DPV 15b-beta-glucuronidase, no intracellular enzyme activity was detected in the beta-glucuronidase assay. Nevertheless, it could not be excluded the possibility that DPV15b-beta-glucuronidase is internalized and localized in cellular structures that were not recovered by the lysis buffer used.

M6P/beta-glucuronidase was normally internalized into the fibroblastic cells as described previously (LeBowitz *et al.,* 2004). DPV3 conjugation on M6P/beta-glucuronidase allowed a 3-fold increasing in enzyme uptake by MPSVII cells. Uptake was also significantly increased with DPV 15b conjugated but it remained lower than with DPV3 conjugated.

The DPV3 peptide therefore allowed the increased intracellular uptake when conjugated with both enzymes (beta-glucuronidase or M6P/beta-glucuronidase).

### II-2-2 Intracellular Localization of Transduced Enzyme

In order to further study the subcellular localization of the internalized beta-glucuronidase enzymes, indirect immunofluorescence staining was performed. MPSVII cells were incubated with or without 50µg/mL of each protein for 4 hours at 37°C. Double immunostaining with anti-beta-glucuronidase antibody and anti-LAMP-1 antibody (a lysosomal marker) clearly revealed that internalized DPV3-beta-glucuronidase was localized in vesicular structures in the cytoplasm (Figure 4, *I*) and colocalized with LAMP-1 lysosomal structures (yellow signal) (Figure 4, *K*). DPV3-beta-glucuronidase conjugates allowed internalization of beta-glucuronidase and targeting of beta-glucuronidase to the cellular site (lysosomes) for optimal activity. Concerning DPV15b-beta-glucuronidase conjugate uptake, we detected a nuclear localization of the enzyme that is in accordance with previous studies showing that DPV15b peptide is a nuclear peptide vector (Figure 4, *M*). In addition, this nuclear localization of beta-glucuronidase uptake could explain the lack of beta-glucuronidase activity in cytoplasm extract of MPSVII cells, which were incubated with DPV 15b-beta-glucuronidase (Figure 3A).

Immunofluorescence experiments were performed to visualize DPV3-beta-glucuronidase internalization but this technique could not be used to visualize the bovine DPV3-M6P/beta-glucuronidase because specific antibodies that cross react with the bovine enzyme are not available. Chromogenic staining of cells incubated for 4 hours with DPV3- or non-M6P/beta-glucuronidase was therefore performed to visualize the internalization and activity of DPV3-M6P/beta-glucuronidase. The 5-bromo-4-chloro-3-indolyl beta-D-glucuronic acid (X-gluc) reagent provided a colorimetric method for the intracellular localization of beta-glucuronidase activity. As shown in figure 5, it was observed a cytoplasmic staining of cells treated with both M6P/beta-glucuronidase and DPV3-M6P/beta-glucuronidase proteins. The intensity of staining is significantly higher in cells incubated with DPV3-M6P-beta-glucuronidase compared to cells incubated with M6P-beta-glucuronidase. This observation was consistent with the previous experiment that showed a three-fold increase in the intracellular activity of DPV3-M6P/beta-glucuronidase internalization compared to M6P/beta-glucuronidase (Figure 3B).

### II-3 In Vitro Characterization of Transduced-Enzyme Biological Activity

Only DPV3 peptide allows an increase of uptake of both enzyme forms (phosphomanosylated and non- phosphomanosylated beta-glucuronidase). Experiments were undertaken to evaluate whether delivery of DPV3-beta-glucuronidase or DPV3-M6P/beta-glucuronidase could induce a decrease of glycosaminoglycan (GAG) in MPSVII cells.

Preliminary studies were initiated to define an analytic method allowing quantifying cellular sulfated GAGs in culture cells. As described in the Material and Methods, sulfated GAGs were extracted from MPSVII and HeLa cells and quantified using a dimethylene blue dye-binding assay. The sulfated GAG content of cell extracts was also analyzed using a qualitative method consisting of agarose gel electrophoresis and toluidine blue staining. The level of sulfated GAGs in MPSVII cells was approximately ten-fold greater than in HeLa cells (Figure 6A). The major polysaccharide extracted from MPSVII cells migrated on agarose gel as a single and homogeneous metachromatic band with a mobility between standards of mammalian dermatan sulfate (DS) and chondroitin sulfate A (CSA). No sulfated GAGs were visualized in the HeLa extract. These results are consistent with the pathology described for deficient beta-glucuronidase cells (GAGs accumulation).

The effect of internalized beta-glucuronidase on the GAG storage process was further investigated by measuring the levels of sulfated GAGs in MPSVII cells incubated with DPV3 conjugated enzyme. The conjugation of DPV3 to M6P/beta-glucuronidase improved significantly the degradation of the intracellular GAGs found in MPSVII cells (about 1.6 fold in each assay; n=3) in comparison with M6P/betaglucuronidase alone or in combination with DPV3 (5µg/ml of protein for 6 hours) (Figure 7).

## Claims

1. A chimeric polypeptide comprising a first domain and a second domain, wherein
the first domain comprises an amino-acid sequence facilitating active transport across a biological membrane by the binding to an aminoglycan, which is selected from the group consisting of a) (XBBBXXBX)n ; b) (XBBXBX)n ; c) (BBXmBBXp)n ; d) (XBBXXBX)n ; e) (BXmBB)n ; f) (BmXX)n or g) an antibody fragment, wherein each B is independently a basic amino acid preferably lysine or arginine; each X is independently a non-basic amino acid preferably hydrophobic amino acid; each m is independently an integer from zero to five; each n is independently an integer between one and ten; and each p is independently an integer between zero to five; and
the second domain comprises the amino acid sequence of a lysosomal enzyme.

2. The chimeric polypeptide according to claim 1, wherein the first domain comprises an amino-acid sequence facilitating active transport across a biological membrane, having a length of more than 4 amino acids, preferably more than 6 amino acids.

3. The chimeric polypeptide according to any one of claims 1 or 2, wherein the first domain comprises an amino-acid sequence facilitating active transport across a biological membrane, having a length of less than 500 amino acids, preferably less than 25 amino acids.

4. The chimeric polypeptide according to any one of claims 1 to 3, wherein each X is independently alanine, glutamic acid, isoleucine, leucine, methionine, phenylalanine, serine, tryptophan, valine or tyrosine.

5. The chimeric polypeptide according to any one of claims 1 to 4, wherein the first domain comprises an amino acid sequence selected from the group comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12.

6. The chimeric polypeptide according to claim 1, wherein the translocation sequence comprises the amino acid sequence (BmXX)n; and wherein B is any basic amino acid; X is any non-basic amino acid; each m is independently a integer between 1 and 10; each n is independently a integer between 1 and 4.

7. The chimeric polypeptide according to claim 6, wherein the translocation sequence comprises the amino acid sequence BBBBBBXXBBBBBBXX; wherein B is any basic amino acid; X is any non-basic amino acid.

8. The chimeric polypeptide according to claim 7, wherein X is selected from the group comprising glutamic acid and serine.

9. The chimeric polypeptide according to claim 8, wherein the translocation sequence comprises SEQ ID NO: 2.

10. The chimeric polypeptide according to any of the previous claims, wherein the lysosomal enzyme has the ability to suppress, prevent the symptoms of a Lysosomal Storage Disorder or rescue a Lysosomal Storage Disorder gene defect.

11. The chimeric polypeptide according to any of the previous claims, wherein the lysosomal enzyme is selected from the group comprising Acid-alpha-1, 4-glucosidase; beta-Galactosidase; beta-Galactosidase A; beta-Hexosaminidase A; beta-Hexosaminidase B; GM₂ Activator Protein; Glucocerebrosidase; Arylsulfatase A; Arylsulfatase B; Galactosylceramidase; Acid Sphigomyelinase; Cholesterol; Acid Ceramidase; Acid Lipase; alpha-L-Iduronidase; Iduronidase Sulfatase; Heparan N-Sulfatase; alpha-N-Acetylglucosaminidase; Acetyl-CoA-Glucosaminide Acetyltransferase; N-Acetylglucosamine-6-Sulfatase; Galactosamine-6-Sulfatase; beta-Glucuronidase; alpha-Mannosidase; beta-Mannosidase; alpha-L-Fucosidase; N-Aspartyl-beta-Glucosaminidase; alpha-Neuraminidase; Lysosomal Protective Protein; alpha-N-Acetyl-Galactosaminidase; N-Acetylglucosamine-1-Phosphotransferase; Cystine Transport Protein; Sialic Acid Transport Protein; Palmitoyl-Protein Thioesterase; Saposins A, B, C or D; and_Cathepsin proteins.

12. The chimeric polypeptide according to claim 11, wherein the lysosomal enzyme is beta-Glucuronidase.

13. The chimeric polypeptide according to any of the previous claims, wherein the lysosomal enzyme is conjugated at the N- or C-terminal end of the translocation sequence.

14. The chimeric polypeptide according to claim 13, wherein the lysosomal enzyme is conjugated to the translocation sequence via a linker.

15. The chimeric polypeptide according to claim 14 wherein the linker is succinimidyl 4-(N-maleimidomethyl)cyclohexane-1-carboxylate (SMCC) or N-maleimidobutylamine.

16. The chimeric polypeptide according to any of claims 13 or 14, wherein both domains are fused by genetic engineering.

17. A polynucleotide comprising a nucleic acid sequence encoding a polypeptide according to claim 16.

18. A use of an amino acid sequence corresponding to a first domain as described in any one of the claims 1 to 9 to transfer substances of interest into lysosomes.

19. The use according to claim 18, wherein the substance of interest is a lysosomal enzyme as described in any one of the claims 11 or 12.

20. A composition comprising a chimeric polypeptide according to any of claims 1 to 16 or a polynucleotide according to claim 17 and optionally a pharmaceutically acceptable carrier.

21. A use of a chimeric polypeptide according to any of claims 1 to 16 or a polynucleotide according to claim 17 for the manufacture of a medicament for treating a lysosomal storage disease.

22. The use according to claim 21, wherein the lysosomal storage disease is Sly Syndrome (MPS VII).
